(19) Europäisches Patentamt
European Patent Office
Office européen des brevets

(11) Publication number: 0 125 701
B1

(12) # EUROPEAN PATENT SPECIFICATION

(45) Date of publication of patent specification: **03.08.88**

(21) Application number: **84105584.1**

(22) Date of filing: **16.05.84**

(51) Int. Cl.[4]: **C 07 C 91/10**, C 07 C 91/04, C 07 C 93/187, C 07 C 93/04, C 07 C 91/14, C 07 C 89/00, A 61 K 31/13, A 61 K 31/645, A 61 K 31/22, C 07 C 119/10, C 07 C 103/78

(54) **Polycyclic aromatic compounds.**

(30) Priority: **17.05.83 GB 8313571**

(43) Date of publication of application:
**21.11.84 Bulletin 84/47**

(45) Publication of the grant of the patent:
**03.08.88 Bulletin 88/31**

(84) Designated Contracting States:
**AT BE CH DE FR GB IT LI LU NL SE**

(56) References cited:
**DE-A-2 362 749**
**US-A-2 865 925**
**US-A-3 198 835**
**ARZNEIMITTELFORSCHUNG, DRUG RESEARCH, vol. 7, 1982, Aulendorf HRABOWSKA et al. "Antitumor activity of 1-nitro-9-amino-acridine derivatives" pages 1013-1016**
**BEILSTEINS "Handbuch der organischen Chemie", 4th edition, vol. 5, supplement 4, 1980 SPRINGER-VERLAG, Berlin, Heidelberg, New York pages 2313, 2314**

(73) Proprietor: **THE WELLCOME FOUNDATION LIMITED**
**183-193 Euston Road**
**London NW1 2BP (GB)**

(72) Inventor: **Bair, Kenneth Walter**
**342 Wesley Drive**
**Chapel Hill North Carolina 27514 (US)**

(74) Representative: **Sandmair, Kurt, Dr. et al**
**Patentanwälte Schwabe, Sandmair, Marx**
**Stuntzstrasse 16**
**D-8000 München 80 (DE)**

(56) References cited:
**CHEMICAL ABSTRACTS, vol. 88, no. 5, January 30, 1978, Columbus, Ohio, USA D.O. SHAH "Synthesis of phenanthroindolizidine derivatives" page 523, abstract no. 38 036w**
**CHEMICAL ABSTRACTS, vol. 76, no. 18, May 1, 1972, Columbus, Ohio, USA R.M. PECK "Mixed bifunctionality. 4. Antitumor activity of alkylating derivatives of polycyclic aromatic hydrocarbons as a function of structure and of vehicle" page 11, abstract no. 107 844z**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 701 B1

(56) References cited:

CHEMICAL ABSTRACTS, vol. 93, no. 19, November 10, 1980, Columbus, Ohio, USA W.C. ALCORN et al. "Reactions of copper(II)halides with aromatic compounds. Part XIII. The mechanism of halogenation of some substituted 9-methylanthracenes" page 631, abstract no. 186 031j

CHEMICAL ABSTRACTS, vol. 95, no. 9, August 31, 1981, Columbus, Ohio, USA T. POETTINGER "Synthesis of (+-)-14-hydroxy-2,3,6-trimethoxy-9-11,12,13,13a,14-hexahydrodibenzo(f,h)pyrrolo(1,2-b)isoquinoline" page 819, abstract no. 81 292h

CHEMICAL ABSTRACTS, vol. 74, no. 15, April 12, 1971, Columbus, Ohio, USA P. BAUMGARTNER et al. "Higher, condensed ring systems. 6. Dehydrogenation of 9,10-dihydrophenanthrenes by N-bromosuccinimide, Synthesis of (23)(2,7)phenanthrenophanetriene" page 424, abstract no. 76 213v

CHEMICAL ABSTRACTS, vol. 75, no. 13, September 27, 1971, Columbus, Ohio, USA M.D. BENTLEY "Nucleophilicity of 2,2,2-trifluoroethanol" page 311, abstract no. 87 977u

CHEMICAL ABSTRACTS, vol. 73, no. 5, August 3, 1970, Columbus, Ohio, USA TETSUO OTSUBO et al. "Synthesis of pyrene derivatives" page 338, abstract no. 25 176g

CHEMICAL ABSTRACTS,vol. 67, no. 21, November 20, 1967, Columbus, Ohio, USA D. COHEN et al. "Reactions of 5,6-chrysenequinodimethane" page 9388, abstract no. 99 901n

**Description**

The present invention relates to polycyclic aromatic alkanol derivatives which have been found to have biocidal activity. More specifically the invention concerns aminoalkanol derivatives containing a polycarbocyclic aromatic ring system, methods for the synthesis thereof, pharmaceutical formulations thereof, and the use thereof as biocidal agents, particularly antitumor agents.

*Gazz. Chim. Ital.* 93, 118 (1963) reports the preparation of 2-phenylmethylamino-2-methyl-1,3-propanediol, but no antitumor activity is disclosed for this compound. Two analogues of nitracrine (1-nitro-9-((3-dimethylaminopropyl)amino)-acridine) containing 2-amino-2-methyl-1,3-propanediol and tris(hydroxymethyl)methylamine groups have shown antitumor activity in murine screening systems (*Arzneim. Forsch./Drug Res.* 3211, 1013 (1982)).

US—PS 2 865 925 discloses N-(2-Hydroxyethyl)-N-(lower-alkyl)-9-aminomethylanthracenes which are described as useful antifibrillatory agents and as intermediates in preparing adrenolytic agents. There is no biocidal, in particular, no antitumor activity described for these compounds. However, the compound N-(2-hydroxyethyl)-N-methyl-9-aminomethylanthracene would fall under the scope of claim 1; this compound is therefore excluded from the present application.

We have now discovered a novel class of polycarbocyclic aromatic alkanol derivatives which have biocidal activity.

Accordingly, in a first aspect, the present invention provides a compound of the formula (I):

$$ArCH_2R^1 \qquad (I)$$

or a monomethyl or monoethyl ether thereof, the compound of formula (I) including its ethers containing no more than 30 carbon atoms in total, or an ester or a salt thereof;

wherein Ar is perylene, fluoranthene, chrysene, pyrene or triphenylene, optionally substituted by one or two substituents, or when Ar is anthracene or phenanthrene optionally substituted by one, two, or three substituents; said substituents containing not more than four carbon atoms in total when taken together being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $(S(O)_nR^2$ wherein n is an integer 0,1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five- or six-membered heterocyclic ring optionally containing one or two additional heteroatoms;

$R^1$ contains not more than eight carbon atoms and is a group

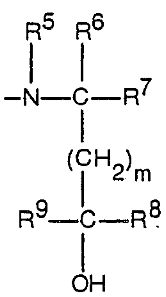

or

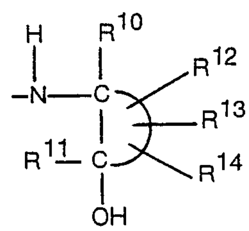

wherein

m is 0 or 1;

$R^5$ is hydrogen or methyl;

$R^6$ and $R^7$ are the same of different and each is hydrogen or $C_{1-3}$ alkyl optionally substituted by hydroxyl;

$R^8$ and $R^9$ are the same or different and each is hydrogen or $C_{1-3}$ alkyl;

—C—C— is a five- or six-membered saturated carbocyclic ring;

$R^{10}$ is hydrogen, methyl or hydroxymethyl;

$R^{11}$, $R^{12}$ and $R^{13}$ are the same of different and each is hydrogen or methyl;

$R^{14}$ is hydrogen, methyl, hydroxy, or hydroxymethyl;

and provided that when Ar is 6-chrysenyl, 3- or 7-fluoranthenyl, 2-triphenylenyl, or 1- or 9-anthracenyl optionally substituted by one or two substituents as hereinbefore defined other than optionally substituted butyl or butoxy; $R^6$ is $C_{1-3}$ alkyl optionally substituted by hydroxy; $R^7$ is hydrogen, $C_{1-3}$ alkyl or hydroxymethyl; $R^8$ to $R^{12}$ and $R^{14}$ are as hereinbefore defined; $R^{13}$ is hydrogen; and m is 0; then $R^5$ is methyl; and the compound, N-(2-hydroxyethyl)-N-methyl-9-aminoethylanthracene being excluded.

The names and numbering systems used for the aromatic ring systems (Ar) are indicated below:

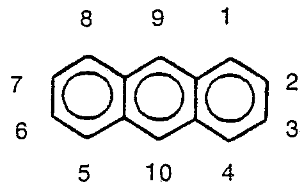

Anthracene

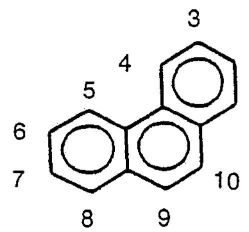

Phenanthrene

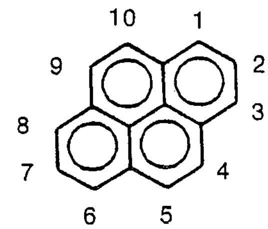

Pyrene

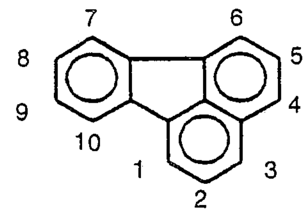

Fluoranthene

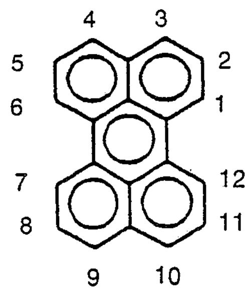

Perylene

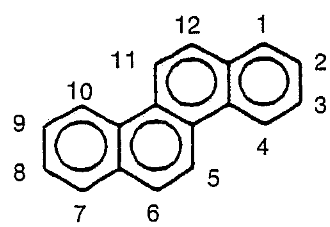

Chrysene

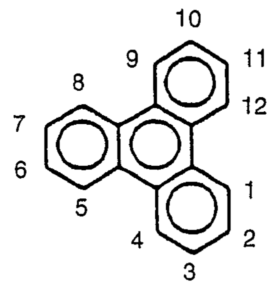

Triphenylene

Suitably $ArCH_2R^1$ or a monomethyl or monethyl ether thereof contains not more than 28 carbon atoms in total.

Ar is suitably 6-chrysenyl, 3- or 7-fluoranthenyl, 2-triphenylenyl, 1- or 9-anthracenyl, 3-perylenyl, 9-phenanthrenyl or 1-pyrenyl.

Suitably m is O and $R^5$ is hydrogen.

Suitably $R^1$ is

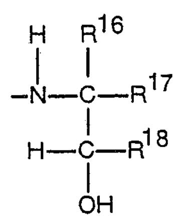

or

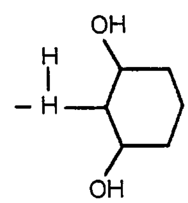

wherein

$R^{16}$ is $CH_2OH$, $CH(CH_3)OH$ or $CH_2CH_2OH$,

$R^{17}$ is hydrogen, $C_{1-3}$ alkyl or $CH_2OH$

$R^{18}$ is hydrogen or methyl;

Suitably $R^{16}$ is $CH_2OH$ or $CH(CH_3)OH$; Suitably $R^{17}$ is hydrogen, methyl, ethyl or $CH_2OH$.

Preferably $R^1$ is

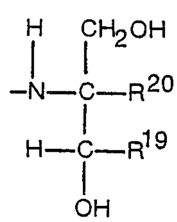

wherein $R^{19}$ is hydrogen or methyl and $R^{20}$ is hydrogen, methyl or ethyl, preferably methyl.

Salts included within the scope of the present invention are those of compound of formula (I) and ethers and esters thereof.

Esters and non-pharmaceutically useful salts of the compounds of the formula (I) are useful intermediates in the preparation and purification of compounds of the formula (I) and pharmaceutically

useful salts thereof, and are therefore within the scope of the present invention. Thus, salts of the compounds of the formula (I) useful in the present invention include but are not limited to those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pantothenic, methanesulfonic, ethanesulfonic, benzenesulfonic, *p*-toluenesulfonic, naphthalene-2-sulfonic, and ascorbic acids, and amino acids such as glycine.

Salts particularly useful as biocidal agents are those that are pharmacologically and pharmaceutically acceptable. Thus, preferred salts include but are not limited to those derived from hydrochloric, methanesulfonic, ethanesulfonic and isethionic acids.

Suitable salts include hydrochlorides, methane- and ethanesulfonates, lactates, citrates and isethionates.

Pharmacologically and pharmaceutically acceptable salts are preferred, particularly those that are soluble in solvents suitable for parenteral administration, for example, hydrochlorides, methanesulfonate and isethionates.

Esters of compounds of formula (I) are derived from acids known to those skilled in the art to be suitable for ester formation, and are conveniently those derived from $C_{1-6}$ alkanoic acids or alkanoic acid derivatives, for example acetic acid, propionic acid, *n*-butyric acid and *iso*-butyric acid. The esters may be formed from all or only some of the hydroxy groups contained in the compounds of formula (I). Specific compounds within the scope of formula (I) include;

2-Methyl-2-((1-pyrenylmethyl)amino)propanol
3-((1-Pyrenylmethyl)amino)propanol
3-Methyl-2-((1-pyrenylmethyl)amino)butanol
2-Ethyl-2-((1-pyrenylmethyl)amino)-1,3-propanediol
2-((1-Pyrenylmethyl)amino)butanol
2-((1-Pyrenylmethyl)amino)propanol
1-((1-pyrenylmethyl)amino)-2-propanol
2-Methyl-2-((1-pyrenylmethyl)amino)-1,3-propanediol
1-((1-Pyrenylmethyl)amino)-2-butanol
2-(*N*-(1-Pyrenylmethyl)methylamino)ethanol
2-Hydroxymethyl-2-((1-pyrenylmethyl)amino)-1,3-propanediol
2-((1-Pyrenylmethyl)amino)-1,3-propanediol
2-(((1-Pyrenylmethyl)amino)methyl-2-propanol
2-((1-Pyrenylmethyl)amino)ethanol
4-((1-Pyrenylmethyl)amino)-2-butanol
2-Methyl-2-((9-phenanthrenylmethyl)amino)-1,3-propanediol
2-Methyl-2-((3-Perylenylmethyl)amino)-1,3-propanediol
2-Methyl-2-((4-phenanthrenylmethyl)amino)-1,3-propanediol
2-Methyl-2-(((4,5,10-trichloro-9-anthracenyl)methyl)amino)-1,3-propanediol
2-(((10-Chloro-2,3-dimethyl-9-anthracenyl)methyl)amino)-2-methyl-1,3-propanediol
2-(((2-*tert*-Butyl-10-chloro-9-anthracenyl)methyl)amino)-2-methyl-1,3-propanediol
2-(((3-*tert*-Butyl-10-chloro-9-anthracenyl)methyl)amino)-2-methyl-1,3-propanediol
2-Methyl-2-((2-phenanthrenylmethyl)amino)-1,3-propanediol
2-Methyl-2-((3-phenanthrenylmethyl)amino)-1,3-propanediol
2-((2-Anthracenylmethyl)amino)-2-methyl-1,3-propanediol
2-Methyl-2-(((2,6,10-trichloro-9-anthracenyl)methyl)amino)-1,3-propanediol
2-(((6-Bromo-1-pyrenyl)methyl)amino)-2-methyl-1,3-propanediol and 2-(((8-Bromo-1-pyrenyl)methyl)-amino)-2-methyl-1,3-propanediol
2-Methyl-2-((4-pyrenylmethyl)amino)-1,3-propanediol
2-((6-Chrysenylmethyl)amino)-2-methyl-1-propanol
2-(((10-Butoxy-9-anthracenyl)methyl)amino)-2-methyl-1,3-propanediol
2-(((10-Butyl-9-anthracenyl)methyl)amino)-2-methyl-1,3-propanediol
2-((3-Fluoranthenylmethyl)amino)-2-methyl-1-propanol
2-((8-Fluoranthenylmethyl)amino)-2-methyl-1,3-propanediol
(+−)(2*R**,3*S**)-2-((1-Pyrenylmethyl)amino)-2-methyl-1,3-butanediol
(+−)(2*R**,3*S**)-2-((1-Phenanthrenylmethyl)amino)-2-methyl-1,3-butanediol
2-(((3-Chloro-8-fluoranthenyl)methyl)amino)-2-methyl-1,3-propanediol and 2-(((4-Chloro-8-fluoranthenyl)methyl)amino)-2-methyl-1,3-propanediol
2-Ethoxymethyl-2-((1-pyrenylmethyl)amino)-1,3-propanediol
2-Ethoxymethyl-2-((9-phenanthrenylmethyl)amino)-1,3-propanediol
2-(((10-Ethoxy-9-phenanthrenyl)methyl)amino)-2-methyl-1,3-propanediol
2-(*N*-((6-Chrysenylmethyl)-*N*-methylamino)-2-methyl-1,3-propanediol
2-((3-Chrysenylmethyl)amino)-2-methyl-1,3-propanediol
2-((2-Chrysenylmethyl)amino)-2-methyl-1,3-propanediol
2-(*N*-(3-Fluoranthenylmethyl)-*N*-methylamino)-2-methyl-1,3-propanediol
3-Methoxy-2-methyl-2-((1-pyrenylmethyl)amino)-1-propanol

3-Methoxy-2-methyl-2-((9-phenanthrenylmethyl)amino)-1-propanol
2-(((3-Ethyl-8-fluoranthenyl)methyl)amino)-2-methyl-1,3-propanediol
1-(((4-Ethyl-8-fluoranthenyl)methyl)amino)-2-methyl-1,3-propanediol
2-(((9-Ethoxy)-1-phenanthrenyl)methyl)amino)-2-methyl-1,3-propanediol
2-((3-Perylenylmethyl)amino)-3-methoxy-2-methyl-1-propanol
(+−)(2*R**,3*S**)-2-((3-Perylenylmethyl)amino)-2-methyl-1,3-butanediol
2-((3-Perylenylmethyl)amino)-2-ethoxymethyl-1,3-propanediol
2-Methyl-2-((9-phenanthrenylmethyl)amino)-1,3-butanediol
(1α,2β,3α)-2-((1-Pyrenylmethyl)amino)-1,3-cyclohexanediol
2-Isopropyl-2-((3-perylenylmethyl)amino)-1,3-propanediol
2-Methyl-2-((3-perylenylmethyl)amino)-1,4-butanediol
(1α,2β,3α)-2-((3-Perylenylmethyl)amino)-1,3-cyclohexanediol
2-(((3-Ethyl-1-pyrenyl)methyl)amino)-2-methyl-1,3-propanediol
2-Isopropyl-2-((9-phenanthrenylmethyl)amino)-1,3-propanediol
(1α,2β,3α)-2-((9-Phenanthrenylmethyl)amino)-1,3-cyclohexanediol
2-Isopropyl-2-((1-pyrenylmethyl)amino)-1,3-propanediol
2-(((8-Ethoxy-1-pyrenyl)methyl)amino)-2-methyl-1,3-propanediol

and ethers, esters, and salts thereof.

The compounds of formula (I) and their ethers, esters, and salts thereof may be prepared by any method known in the art for the preparation of compounds of analogous structure. Thus, the compounds of formula (I) may, for example, be prepared by any of the methods defined below.

1. The reduction of a compound of formula (II)

($R^1$ when $R^5$ = H)

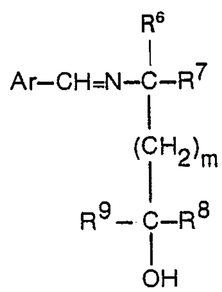

(II)

Wherein $R^1$—$R^4$ are as hereinbefore defined or an appropriately protected derivative thereof followed by deprotection where appropriate.

The conditions and reagents for such a reaction are well known to those skilled in the art, and any such conditions/reagents may be employed. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, *Advance Organic Chemistry,* 2nd ed., pages 819—820, McGraw Hill, New York, 1977. The reduction is suitably carried out with the compound of formula (II) in solution in an inert solvent or mixture of solvents compatible with the reducing agent, at a non-extreme temperature, for example, between 0° and 80°C, conveniently at room temperature.

In the case of lithium aluminum hydride and the like reagents, suitable solvents include ethers (for example tetrahydrofuran, diethyl ether and 1,2-dimethoxyethane) optionally in the presence of a hydrocarbon cosolvent (for example toluene, benzene or hexane).

In the case of sodium borohydride and the like reagents, suitable solvents include alcohols (for example ethanol, methanol or isopropanol) optionally in the presence of a hydrocarbon cosolvent (for example toluene, benzene or hexane) or an ether cosolvent (for example diethylether or tetrahydrofuran).

In the case of sodium cyanoborohydride and like reagents, suitable solvents include those described for sodium borohydride and in the presence of an acid conveniently glacial acetic acid or ethanolic hydrochloric acid as outlined in, for example, R. Hutchins *et al., Organic Preparations and Procedures International 11,* 201 (1979).

In the case of catalytic hydrogenation, suitable solvents include alcohols (for example methanol and ethanol) optionally in the presence of a hydrocarbon cosolvent (for example toluene or benzene) or ether cosolvent (for example diethyl ether or tetrahydrofuran) optionally in the presence of an acid (for example glacial acetic acid or ethanolic hydrochloric acid) or in glacial acetic acid.

Protected derivatives of compounds of formula (II) are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.

It is often convenient not to isolate the compound of the formula (II) but to react a compound of the formula (III) with a compound of the formula (IV):

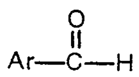

(III)

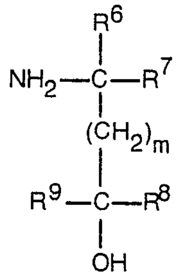

(IV)

(where $R^5 = H$)

wherein Ar and $R^1$—$R^4$ are as defined in (I) except that $R^5$=H, and reduce the compound of the formula (II) so formed *in situ.* The reaction of the compounds of the formulae (III) and (IV) is again suitably carried out using conditions and reagents which are well known to those skilled in the art, for example in the presence of an acid, such as a sulfonic acid, i.e. *p*-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol. Alternatively, (II) formed under equilibrium conditions in appropriate solvents can be reduced *in situ* with an appropriate reducing agent, suitably sodium cyanoborohydride. The compound of formula (III) may be in the form of a protected aldehyde, for example an acetal, which liberates the aldehyde function under the reaction conditions.

In turn, a compound of formula (III) can be synthesized by reacting the appropriate polycyclic aromatic hydrocarbon with a formylating agent such as that generated by the reaction between $SnCl_4$ and $Cl_2CHOCH_3$ or equivalent reagents, for example, according to the method of A. Reiche *et al., Chem. Ber.* 93, 88 (1960), or with other standard formylating reagents/procedures known to the art, for example, the Gatterman-Koch reaction ($CO/HCl/AlCl_3/CuCl$), the Gatterman reaction ($HCN/HCl/ZnCl_2$), and the Vilsmeier reaction ($POC_3$/PhN(Me)CHO or $POCl_3/Me_2NCHO$) (J. March, *vide supra* pages 494—497).

The compounds of the formula (III) may also be prepared from an appropriate polycyclic aromatic hydrocarbon substituted by a suitable functional group such as $CH_2OH$, $CHBr_2$, $CH_3$, $COCH_3$, COOH, or CN, and converting this functional group to an aldehyde group by methods well known to those skilled in the art.

Where the polycyclic aromatic ring bears *substituents*, the compound of formula (III) may be prepared by a variety of methods known in the art of organic chemistry depending on the nature of the substituent on the polycyclic ring. For example, if the substituent(s) is a halogen, the starting materials may be prepared by direct treatment of the polycyclic aromatic hydrocarbon with a halogenating agent (e.g. $Cl_2$, $Br_2$, or $SO_2Cl_2$) or indirectly by such routes as the Sandmeyer reaction (H. H. Hodgson, *Chem. Rev.* 40, 251 (1947). If the substituent(s) is alkyl, the polycyclic aromatic hydrocarbon may be reacted with the appropriate reagents under Friedel-Crafts reaction conditions (G. A. Olah, *Friedel Crafts and Related Reactions,* Vols. 1—3, Interscience, New York, NY, 1963—1965).

The compounds of the formula (IV) also may be prepared by methods known in the art, for example, by the reaction of compound $NO_2CH_2R^2$ with an appropriate aldehyde, conveniently acetaldehyde or formaldehyde (as in B. M. Vanderbilt and H. B. Hass, *Ind. Eng. Chem.* 32, 34 (1940)) followed by reduction (as outlined in J. March, *vide supra,* pages 1125—1126, conveniently by hydrogen and a metal catalyst (for example, a platinum containing catalyst) in an appropriate solvent, conveniently glacial acetic acid.

2. The reduction of a compound of the formula (V)

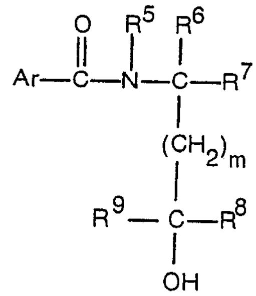

(V)

wherein Ar and $R^1$—$R^4$ are as hereinbefore defined and the hydroxy groups are optionally protected, followed by deprotection of the hydroxy groups where appropriate. The reduction may be carried out by standard reducing agents known for carrying out this type of reduction (as outlined in J. March, *vide supra,* page 1122), for example, a hydride reagent such as lithium aluminium hydride in an inert solvent, such as

an ether, i.e. tetrahydrofuran, at a non-extreme temperature, for example, at between 0° and 100°C and conveniently at the reflux temperature of the ether.

The compound of the formula (V) may be formed by the reaction of the appropriate acid (ArCOOH) or a suitable reactive acid derivative thereof as outlined in J. March, *vide supra,* pages 382—390) for example, an acid halide in an inert solvent, with an amine of the formula (IV) in which the hydroxy groups are optionally protected, for example, when the compound of the formula (IV) is a diol, by an isopropylidene group. The compound of the formula (V) so formed is suitably reduced *in situ* and deprotected if necessary to give a compound of formula (I). The compounds of the formula ArCOOH can be prepared by methods well known to those skilled in the art.

3. The reaction of a compound $ArCH_2L$ (wherein Ar is as hereinbefore defined and L is a leaving group), with a compound of the formula (IV) as hereinbefore defined. Suitable leaving groups are those defined by J. March, *vide supra* pages 325—331, and include halogens such as chlorine or bromine and sulfonic acid derivatives such as *p*-toluenesulfonate. The reaction is suitably carried out in an appropriate solvent, such as a dipolar aprotic solvent or alcohol at a non-extreme temperature, for example 50—100°. The compounds of the formula $ArCH_2L$ can be prepared by methods well known to those skilled in the art.

There is therefore provided, as a further aspect of the invention, a method for the preparation of a compound of formula (I) comprising any method known for the preparation of analogous compounds, in particular those methods defined in (1) to (3) hereinabove.

The compounds of this invention have biocidal activity, e.g. are toxic to certain living cells which are detrimental to mammals, for example pathogenic organisms and tumour cells.

This toxicity to pathogenic organisms has been demonstrated by activity against viruses (e.g. *Herpes simplex* I/vero), fungi (e.g. *Candida albicans*), protozoa (e.g. *Eimeria tenella* and *Trichomonas vaginalis*), bacteria (e.g. *Mycoplasma smegmatis* and *Streptococcus pyogenes*), and helminths (e.g. *Nippostrongylus brasiliensis* and *Brugia pahangi*). The antitumour activity of compounds of formula I has been demonstrated in a number of recognized screens and primarily by activity against ascitic P388/O leukaemia.

Preferred compounds of the formula (I) are those which have antitumour activity. The activity against ascitic tumours, including P388/O, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/O, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour activity in man (A. Goldin *et al.* in *Methods in Cancer Research* ed. V. T. DeVita Jr. and H. Busch, *16*, 165, Academic Press, N.Y. 1979).

There are sublines of P388/0 which have been made resistant to the following clinically useful agents: cytosine arabinoside, doxorubicin, cyclophosphamide, *L*-phenylalanine mustard, methotrexate, 5-fluorouracil, actinomycin D, *cis*-platin and bis-chloroethylnitrosourea. Compounds of this invention show potent activity against these drug-resistant tumours using the procedure for P388/O above.

Compounds of formula (I) have also been found to be active against human tumour cells in primary cultures of lung, ovary, breast, anal, melanoma, unknown primary, gastric, pancreatic, mesothelioma, myeloma, and colon cancer. (As used herein "cancer" is to be taken as synonymous with "malignant tumour" or more generally "tumour" unless otherwise noted.) This is a procedure in which the prevention of tumour cell colony formation, i.e. tumour cell replication, by a drug has been shown to correlate with clinical antitumour activity in man (D. D. Von Hoff *et al., Cancer Chemotherapy* and *Pharmacology* 6, 265 (1980); S. Salmon and D. D. Von Hoff, *Seminars* in *Oncology,* 8, 377 (1981)).

Compounds of formula I which have been found to have antitumour activity intercalate *in vitro* with DNA (this property is determined by viscometric methods using the procedure of W. D. Wilson *et al., Nucleic Acids Research* 4, 2697 (1954)) and a log P as calculated by the method of C. Hansch and A. Leo in *Substituent Constants for Correlation Analysis in Chemistry and Biology,* John Wiley and Sons, New York, 1979, lying in the range between −2.0 and +2.5.

As has been described above, the compounds of the present invention are useful for the treatment of tumours. The invention thus further provides a method for preparation of a medicament for the treatment of tumors in animals, including mammals, especially humans which comprises the administration of a clinically useful amount of compound of formula (I) in a pharmaceutically useful form, once or several times a day or other appropriate schedule, orally, rectally, parenterally or applied topically.

In addition, there is provided as a further, or alternative, aspect of the invention, a compound of formula (I) for use in therapy, for example as an antitumour agent.

The amount of compound of formula (I) required to be effective as a biocidal agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be

administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, *e.g.* two to six times per day or by intravenous infusion for selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical formulation. Formulations of the present invention, for medical use, comprise an active compound together with one or more pharmaceutically acceptable carriers thereof and optionally other therapeutical ingedients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester, or pharmaceutically acceptable salt thereof, and a pharmaceutically acceptable carrier therefor.

Whilst the antitumour activity of the compounds of formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product into desired formulations.

Formulations of the present invention suitable for oral adminstration may be presented as discrete units such as capsules, cachets, tablets or lozenzes, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar, for example sucrose, to which may also be added any accessory ingredients. Such accessory ingredient(s) may include flavourings, an agent to retard crystallization of the sugar or an agent to increase the solubility of any other ingredient, such as a polyhydric alcohol for example glycerol or sorbitol.

Formulations for rectal adminstration may be presented as a suppository with a conventional carrier such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

General Comments

All solvents were reagent grade and used without further purification with the following exceptions. Tetrahydrofuran (THF) was dried by distillation from Na/K alloy under nitrogen ($N_2$) and used immediately.

Toluene ($PhCH_3$) was distilled from $CaH_2$ under $N_2$ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plugs of $SiO_2$ used for purifications were "flash chromatography" silica gel (Merck & Co., Inc., Merck Chemical Division, Rahway, NJ, 07065 silica gel 60, 230—400 mesh). An appropriate volume sintered glass funnel was filled approximately ¾ full with the $SiO_2$ and packed evenly by tapping the outside of the funnel. A piece of filter paper was then placed on top of the $SiO_2$ and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents, and elemental analyses (all elements analyzing within a difference of ½0.4% of the expected value). Any changes to the procedure such as solvent, reaction temperature, reaction time, or workup are noted.

NMR ($^1H$, $^{13}C$), IR, MS data of all new products were consistent with the expected and proposed structures. The positions assigned to structural isomers were unequivocally determined by a number of NMR techniques. All final products were dried in a vacuum oven at 20 mm Hg pressure at the temperature indicated overnight (12—16 h). All temperatures are in degrees Celsius. Other abbreviations that were used: room temperature (RT), absolute (abs.), round bottom flask (RB flask), minutes (min), hours (h).

Example 1

2-Methyl-2-((1-pyrenylmethyl)amino)-1,3-propanediol

1A. 2-Methyl-2-((1-pyrenylmethyl)amino)-1,3-propanediol hydrochloride

To a 2 L Erlenmeyer flask was added 1-pyrenecarbaldehyde (Aldrich Chemical Co., Milwaukee, WI, 53201, 115.1 g, 0.5 mol), 2-amino-2-methyl-1,3-propanediol (Aldrich, 52.57 g, 0.5 mol), *p*-toluenesulfonic acid'$H_2O$ (Eastman Kodak Co., Rochester, NY, 14650, 1 g, 5 mmol), and PhCH3 (500 mL). The mixture was warmed to reflux for a few minutes and $H_2O$ (α10 mL) was driven off. The resulting yellow solution was allowed to cool to RT, diluted with abs. EtOH (500 mL) and stirred overnight. $NaBH_3CN$ (Aldrich, 95%, 15.71 g, 0.25 mol) was added to the reaction. After the $NaBH_3CN$ dissolved, an indicator (bromocresol green, Eastman, 5 mg) was added. To the resulting blue solution was added 5 drops of 1 M ethanolic HCl every 15 min. After 3 days the indicator turned green then yellow and a voluminous white precipitate was present in the flask. To the flask was then added 1M ethanolic HCl (α50 mL). The reaction was diluted to 4 L with abs. $Et_2O$ and stirred for 1 h. The precipitate was then collected by filtration through a medium porosity glass fritted funnel, washed with $Et_2O$ (2 L), and pressed dry. The filter cake was washed thoroughly with 20% HCl (5 × 250 mL), pressed dry and then washed with $CH_2Cl_2$ (4 × 500 mL), pressed and sucked dry. The white solid was dried at 60– overnight. The off-white crude material (149 g) was dissolved in hot $H_2O$ (2.5 L) containing 20 mL of conc. HCl and and decolorized with 20 g of Calgon (trademark of Calgon Corp., a subsidiary of Merck & Co., Pittsburgh, PA, 15230) brand of activated charcoal, filtered through a pad of Celite (trademark of Johns Manville Co., Denver, CO, 80110) brand of filter-aid to give a total of 2.8 L of clear colorless solution. The volume of the solution was reduced to 1.8 L. The solution was allowed to cool to RT, and then was placed in a refrigerator overnight. The white solid which formed overnight was collected by filtration, dried overnight at 60–, and recrystallized from EtOH/$Et_2O$ to give 104 g (56%) of 2-methyl-2-((1-pyrenylmethyl)amino)-1,3-propanediol hydrochloride mp 231—230–, (C, H, Cl, N).

1B. 2-Methyl-2-((1-pyrenylmethyl)amino)-1,3-propanediol methanesulfonate

To a solution of 1A (35.58 g, 0.1 mol) in $CH_3OH$ (500 mL) was added 1N NaOH (110 mL) dropwise over 15 min. The mixture became hard to stir and was diluted with $H_2O$ (1.5 L). The white solid which formed was filtered and washed successively with warm $H_2O$ (3 × 500 mL), then $CH_3OH$ (200 mL), then $Et_2O$ (3 × 500 mL). It was then dried overnight in a vacuum. Final yield of material was 25.4 g (79.5%) which was used without further purification.

The free base (17.5 g, 54.8 mmol) was suspended in $CH_3OH$, cooled, and treated slowly with methanesulfonic acid (Aldrich, 5.27 g, 3.56 mL, 54.8 mmol). The mixture was stirred at RT for 10 min, warmed to reflux, filtered through a sintered glass funnel, then diluted with $Et_2O$ (3.5 L). An oil formed which then crystallized within a few minutes. The solid was then filtered and recrystallized 2× from $CH_3OH$/$Et_2O$ to give after drying 19.1 g (84.0%) of 2-methyl-2-((1-pyrenylmethyl)amino)-1,3-propanediol methanesulfonate mp 184—186–, (C, H, N, S).

Example 2

2-((6-Chrysenylmethyl)amino)-2-methylpropanol

2A. 6-Chrysenecarbaldehyde

A 5 L 3-neck flask fitted with overhead mechanical stirrer, thermometer, condenser and $N_2$ line was charged with chrysene (Eastman, 100 g, 0.438 mol) and *o*-dichlorobenzene (2.5 L). The liquid was warmed until all the large chunks of solid dissolved (80°) and then cooled quickly to give finely divided crystals. After

further cooling with a salt-ice bath to 5°, $SnCl_4$ (Aldrich, 98%, 228.2 g, 0.876 mol, 102.4 mL) was added in one portion. No temeprature change occurred. The pot temperature was kept below 5°, and 1,1-dichloromethylmethylether (Aldrich, 70.48 g, 0.613 mol, 55.45 mL) was added dropwise over 1 h. The resulting suspension was warmed slowly to 40° over 2 h and further stirred for 16 h. Considerable HCl gas evolution occurred during the warming and the early part of the reaction at 40°. The reaction mixture was then cooled to 10° and hydrolysed by careful addition of cold $H_2O$ (1 L). After 4 h, the layers were separated and the organic layer filtered, dried with ($Na_2SO_4$) (Mallinckrodt Co., St. Louis, MO, 63147, 100 g) and filtered again. The clear yellow solution was split into 2 portions and each passed through a plug of $SiO_2$ (500 g) using $PhCH_3$ as the eluting solvent with 500 mL fractions. This chromatography separated unreacted chrysene (3 g) from the aldehyde and a more polar product. Fractions containing the aldehyde were combined and the $PhCH_3$ removed. Crystals formed during this process and were removed periodically by filtration. After drying (at 60°) the yield of 6-chrysencarbaldehyde was 89.46 g (79.7%) mp 167—169°, (C, H), (lit. 168°, N.P. Buu Hoi, J.—P. Hoeffinger, and P. Jacquignon, *Bull. Soc. Chim. Fr.* 3808 (1968)).

2B. 2-((6-Chrysenylmethyl)amino)-2-methylpropanol hydrochloride

Using the reductive amination procedure described in 1A, 6-chrysenecarbaldehyde (1A) and 2-methyl-2-aminopropanol (Aldrich) gave 2-((6-chryenylmethyl)amino)-2-methyl-1-propanol hydrochloride mp 275—277° (dec), ($CH_3OH/Et_2O$), (C, H, Cl, N).

Example 3

2-Methyl-2-((1-pyrenylmethyl)amino)propanol

3A. 2-Methyl-2-((1-pyrenylmethyl)amino)propanol hydrochloride

Using the reductive amination procedure outlined in 1A, 1-pyrenecarbaldehyde (Aldrich) and 2-methyl-2-amino-1-propanol (Aldrich) gave 2-methyl-2-((1-pyrenylmethyl)amino)propanol hydrochloride mp 253.5—254° (dec), (95% EtOH), (C, H, Cl, N).

3B 2-Methyl-2-((1-pyrenylmethyl)amino)propanol

Using the first part of the method in 1B, 3A gave 2-methyl-2((1-pyrenylmethyl)amino)propanol free base mp 135—136–, (95% EtOH), (C, H, N).

Example 4

3-((1-pyrenylmethyl)amino)propanol hydrochloride

Using the reductive amination procedure outlined in 1A, 1-pyrenecarbaldehyde (Aldrich) and 3-amino-1-propanol (Aldrich) gave 3-((1-pyrenylmethyl)amino)propanol hydrochloride mp 194—195° (dec), ($EtOH/Et_2O$), (C, H, Cl, N).

Example 5

3-Methyl-2-((1-pyrenylmethyl)amino)butanol hydrochloride

Using the reductive amination procedure outlined in 1A, 1-pyrenecarbaldehyde (Aldrich) and 3-methyl-2-aminobutanol (Aldrich) gave 3-methyl-2-((1-pyrenylmethyl)amino)butanol hydrochloride mp 246—248° (dec), (95% EtOH), (C, H, Cl, N).

Example 6

2-Ethyl-2-((1-pyrenylmethyl)amino)-1,3-propanediol

6A. 2-Ethyl-2-((1-pyrenylmethyl)amino)-1,3-propanediol hydrochloride

Using the reductive amination procedure outlined in 1A, 1-pyrenecarbaldehyde (Aldrich) and 2-amino-2-ethyl-1,3-propanediol (Aldrich) gave 2-ethyl-2-((1-pyrenylmethyl)amino)-1,3-propanediol hydrochloride mp 225—226.5° (dec), ($EtOH/Et_2O$), (C, H, Cl, N).

6B. 2-Ethyl-2((1-pyrenylmethyl)amino)-1,3-propanediol methanesulfonate

Using the procedure described in 1B, 6A gave 2-ethyl-2-((1-pyrenylmethyl)amino)-1,3-propanediol methanesulfonate mp 200—201°, ($CH_3OH/Et_2O$), (C, H, N, S).

Example 7

2-((1-Pyrenylmethyl)amino)butanol hydrochloride

Using the reductive amination procedure described in 1A, 1-pyrenecarbaldehyde (Aldrich) and 2-amino-1-butanol (Aldrich) gave 2-((1-pyrenylmethyl)amino)butanol hydrochloride mp 224—225.5 (dec), ($EtOH/Et_2O$), (C, H, Cl, N).

Example 8

2-((1-Pyrenylmethyl)amino)propanol hydrochloride

Using the reductive amination procedure outlined in 1A, 1-pyrenecarbaldehyde (Aldrich) and 2-amino-1-propanol (Aldrich) gave 2-((1-pyrenylmethyl)amino)propanol hydrochloride mp 230—232° (dec), ($EtOH/Et_2O$), (C, H, Cl, N).

Example 9

1-((1-pyrenylmethyl)amino)-2-propanol hydrochloride

Using the reductive amination procedure outlined in 1A, 1-pyrenecarbaldehyde (Aldrich) and *dl*-1-amino-2-propanol (Aldrich) gave 1-((1-pyrenylmethyl)amino)-2-propanol hydrochloride mp 217—218° (dec), (abs, EtOH), (C, H, Cl, N).

Example 10

1-(((1-Pyrenylmethyl)amino)methyl)propanol hydrochloride

Using the procedure outlined in 1A, 1-pyrenecarbaldehyde (Aldrich) and 1-amino-2-butanol (Aldrich) gave 1-(((1-pyrenylmethyl)amino)methyl)propanol hydrochloride, mp 237—238° (dec), (abs. EtOH), (C, H, Cl, N).

Example 11

2-(N-(1-(Pyrenylmethyl)methylamino)ethanol hydrochloride

Using the reductive amination procedure outlined in 1A, 1-pyrenecarbaldehyde (Aldrich) and *N*-methyl-2-aminoethanol (Aldrich) gave 2-(*N*-(1-pyrenylmethyl)methylamino)ethanol hydrochloride mp 244—246° (dec), (95% EtOH), (C, H, Cl, N).

Example 12

2-Hydroxymethyl-2-((1-pyrenylmethyl)amino)-1,3-propanediol hydrochloride

Using the reductive amination procedure outlined in 1A, 1-pyrenecarbaldehyde (Aldrich) and tris (hydroxymethyl)aminomethane (Aldrich) gave 2-hydroxymethyl-2-((1-pyrenylmethyl)amino)-1,3-propanediol hydrochloride mp 232—234° (dec), ($CH_3OH/Et_2O$), (C, H, Cl, N).

Example 13

2-((1-Pyrenylmethyl)amino)-1,3-propanediol hydrochloride

Using the reductive amination procedure outlined in 1A, 1-pyrenecarbaldehyde (Aldrich) and 2-amino-1,3-propanediol (Sigma Chemical Company, St. Louis, MO, 63178) gave 2-((1-pyrenylmethyl)amino)-1,3-propanediol hydrochloride mp 211—213° (dec), ($CH_3OH/Et_2O$), (C, H, Cl, N).

Example 14

2-(((1-Pyrenylmethyl)amino)methyl)-2-propanol hydrochloride

Using the reductive amination procedure outlined in 1A, 1-pyrenecarbaldehyde (Aldrich) and 1-amino-2-methyl-2-propanol (Aldrich) gave 2-(((1-pyrenylmethyl)amino)methyl)-2-propanol hydrochloride, mp 225—226.5° (dec), (abs. EtOH), (C, H, Cl, N).

Example 15

2-((1-Pyrenylmethyl)amino)ethanol hydrochloride

Using the reductive amination procedure outlined in 1A, 1-pyrenecarbaldehyde (Aldrich) and 2-aminoethanol (Aldrich) gave 2-((1-pyrenylmethyl)amino)ethanol hydrochloride mp 202—203° (dec), (95% EtOH) (C, H, Cl, N).

Example 16

4-((1-Pyrenylmethyl)amino)-2-butanol hydrochloride

Using the reductive amination procedure outlined in 1A, 1-pyrenecarbaldehyde (Aldrich) and 4-amino-2-butanol (Aldrich) gave 4-((1-pyrenylmethyl)amino)-2-butanol hydrochloride mp 204—206°, ($EtOH/Et_2O$), (C, H, Cl, N).

Example 17

2-Methyl-2-((9-phenanthrenylmethyl)amino)-1,3-propanediol hydrochloride

Using the reductive amination procedure outlined in 1A, 9-phenanthrenecarbaldehyde (Aldrich) and 2-amino-2-methyl-1,3-propanediol (Aldrich) gave 2-methyl 2-((9-phenanthrenylmethyl)amino)-1,3-propanediol hydrochloride mp 144—146°, ($EtOH/Et_2O$), (C, H, Cl, N).

Example 18

2-Methyl-2-((3-perylenylmethyl)amino)-1,3-propanediol

18A. 3-Perylenecarbaldehyde

Using the formylation procedure outlined in 2A, perylene (Aldrich) gave 3-perylenecarbaldehyde mp 223—233°, ($PhCH_3$), (C, H), lit. mp 236° (darkens 1230°), N. P. Buu-Hoi and C. T. Long, *Recueil* 75 1121 (1956)).

18B. 2-Methyl-2-((3-perylenylmethyl)amino-1,3-propanediol hydrochloride

Using the reductive amination procedure outlined in 1A, 3-perylenecarbaldehyde and 2-amino-2-methyl-1,3-propanediol (Aldrich) gave 2-methyl-2-((3-perylenylmethyl)amino)-1,3-propanediol hydrochloride$\cdot\frac{1}{2}H_2O$ mp 231—232° (dec), ($CH_3OH/Et_2O$), (C, H, Cl, N).

18C. 2-Methyl-2-((3-perylenylmethyl)amino-1,3-propanediol methanesulfonate

To a 1 L 3-neck flask equipped with magnetic stirring bar, condenser, Dean-Stark trap and $N_2$ bubbler was added perylene-3-carbaldehyde (18A, 10.0 g, 35.67 mmol), 2-amino-2-methyl-1,3-propanediol (Aldrich, 7.36 g, 70 mmol), *p*-toluenesulfonic acid (Fisher, 0.1 g) and $PhCH_3$ (500 mL). The dark brown mixture was refluxed for 2 h with azeotropic removal of $H_2O$. Most of $PhCH_3$ was then distilled from the mixture (except 100 mL). This was cooled and diluted to 300 mL with abs. EtOH. $NaBH_4$ (MCB, 2.64 g, 70 mmol) was then added to the flask. The solid in the flask dissolved partially and was further diluted with $PhCH_3$ (200 mL). The mixture was then stirred at RT for 3 h. $H_2O$ (10 mL) was then added to the flask.

Solvent was then removed from the mixture by rotary evaporation to give a dark brown solid. This was shaken with $H_2O$ (500 ml) several times and finally filtered. The crude solid was dissolved in a mixture of $PhCH_3$ and abs. EtOH (1:1, 200 mL) and treated with $CH_3SO_3H$ (Aldrich, 3 mL). The solution was then filtered and diluted with $Et_2O$ to give 8.08 g (48.7%) of 2-methyl-2-((3-perylenylmethyl)amino-1,3-propanediol methanesulfonate mp 211—213– (dec), (C, H, N, S). In some of the analogous procedures following, ethanolic HCl was used instead of methanesulfonic acid to give the corresponding hydrochloride salt.

## Example 19

2-Methyl-2-((4,5,10-trichloro-9-anthracenyl)methyl)-1,3-propanediol

19A. 4,5,10-Trichloro-9-anthracenecarbaldehyde

Using the procedure of V. I. Rogovik *et al., Zh. Org. Khim.* 3 1315 (1967), and with the modification that the reaction was worked up after heating for 3 h at 95°, 1,8-dichloroanthraquinone (Aldrich, 22 g. 0.08 mol) yielded an impure solid that was purified by preparative HPLC usig $PhCH_3$ as the eluting solvent to give 5.29 g (21%) of 4,5,10-trichloro-9-anthracenecarbaldehyde mp 118—120°, ($PhCH_3$), (C, H, C). Almost no product can be isolated using the heating regimen in the reference procedure.

19B. 2-Methyl-2-((4,5,10-Trichloro-9-anthracenyl)methyl)-1,3-propanediol hydrochloride

Using the reductive amination procedure outlined in 1A, 4, 5, 10-trichloroanthracene-10-carbaldehyde (19A) and 2-amino-2-methyl-1,3-propanediol (Aldrich) gave 2-methyl-2-((4,5,10-trichloro-9-anthracenyl)methyl)-1,3-propanediol hydrochloride mp 254—255° (dec), ($EtOH/Et_2O$), (C, H, Cl, N).

## Example 20

2-(((10-Chloro-2,3-dimethyl-9-anthracenyl)methyl)amino)-2-methyl-1,3-propanediol

20A. 10-Chloro-2,3-dimethyl-9-anthracenecarbaldehyde

2,3-Dimethylanthraquinone, prepared by the procedure of C. F. Allen and A. Bell, *Org. Syn. Coll. Vol.* III, 310 (1955), was treated with $Fe/POCl_3/DMF$ using the procedure of V. I. Rogovik *et al., Zh. Org. Khim.* 3 1315 (1967) to give 10-chloro-2,3-dimethyl-9-anthracenecarbaldehyde mp 150—153°, ($PhCH_3/MeOH$), (C, H, Cl).

20B. 2-(((10-Chloro-2,3-dimethyl-9-anthracenyl)methyl)amino)-2-methyl-1,3-propanediol hydrochloride

Using the reductive amination procedure outlined in 1A, 10-chloro-2,3-dimethyl-9-anthracenecarbaldehyde and 2-methyl-2-amino-1,3-propanediol (Aldrich) gave 2-((10-chloro-2,3-dimethyl-9-anthracenylmethyl)amino-2-methyl-1,3-propanediol hydrochloride mp 251—252° (dec), ($CH_3OH/ET_2O$), (C, H, Cl, N).

## Example 21

2-(((-tert-Butyl-10-chloro-9-anthracenyl)methyl)amino)-2-methyl-1,3-propanediol

21A. 2-tert-Butyl-10-chloro-9-anthracenecarbaldehyde and

21B. 3-tert-butyl-10-chloro-9-anthracenecarbaldehydes

2-*tert*-Butylanthraquinone (Chemical Dynamics Corporation, P.O. Box 395, 3001 Hadley Road, South Plainfield, NJ 07080) was reductively formylated using the procedure of V. I. Rogovik *et al., Zh. Org. Khim.* 3 1315 (1967) to give a mixture (α1:1) of *2-* and 3-*tert*-butyl-10-chloro-9-anthracenecarbaldehydes, which were separated by preparative HPLC using the shave/recycle technique to obtain 2-*tert*-butyl-10-chloro-9-anthracenecarbaldehyde mp 126—129°, ($PhCH_3/CH_3OH$), (C, H, Cl), and 3-*tert*-butyl-10-chloro-9-anthracenecarbaldehyde mp 143—147°, ($Ph,CH_3/CH_3OH$), (C, H, Cl).

21C. 2-tert-Butyl-10-chloro-9-anthracenylmethyl)amino)-2-methyl-1,3-propanediol hydrochloride 1/4 $H_2O$

Using the reductive amination procedure described in 1A, 2-*tert*-butyl-10-chloro-9-anthracenecarbaldehyde (21B) and 2-amino-2-methyl-1,3-propanediol (Aldrich) gave 2-(((2-tert-butyl-10-chloro-9-anthracenyl)methyl)amino)-2-methyl-1,3-propanediol hydrochloride ·β$H_2O$ mp 249—250° (dec), ($EtOH/Et_2O$), (C, H, Cl, N).

## Example 22

2-(((3-tert-Butyl-10-chloro-9-anthracenyl)methyl)amino)-2-methyl-1,3-propanediol hydrochloride

Using the reductive amination procedure outlined in 1A, 3-*tert*-butyl-10-chloro-9-anthracenecarbaldehyde (21A) and 2-methyl-2-amino-1,3-propanediol (Aldrich) gave 2-(((3-tert-butyl-10-chloro-9-anthracenyl)methyl)amino)-2-methyl-1,3-propanediol hydrochloride mp 244—245° (dec)(*i*-PrOH/$Et_2O$), (C, H, Cl, N).

Example 23

2-Methyl-2-((2-phenanthrenylmethyl)amino)-1,3-propanediol

23A. 2-Phenanthrenecarbaldehyde

2-Acetylphenathrene (Aldrich) was converted to the corresponding acid by the method of E. Mosettig and J. van de Kamp, *J. Amer. Chem. Soc.* 52 3704, (1930). The acid was then converted to the corresponding acid chloride by the method of E. Mosettig and J. van de Kamp, *J. Amer. Chem. Soc.* 55, 2995 (1933).

To a solution of the crude acid chloride (10.66 g, 45 mmol) in 250 mL of acetone (degassed with $N_2$) was added $Cu(PPh_3)_2BH_4$ (Fluka Chemical Corp., 255 Oser Ave., Hauppauge, NY, 11787, 27.1 g, 45 mmol) as a solid. The reaction mixture was then stirred overnight. The reaction was then filtered, and the solvent removed by rotary evaporation. Further inorganic material was removed by partially dissolving the solid in dry $PhCH_3$ and filtration. The $PhCH_3$ was subsequently removed by rotary evaporation. The solid bisulfite complex of the aldehyde was formed by adding satd. $NaHSO_3$ (150 mL) to the aldehyde and stirring vigorously for 5 h. The complex was removed by filtration and digested in 1N NaOH. The mixture was extracted one with $Et_2O$ (IL). The organic layer was dried ($MgSO_4$), filtered, and the solvent removed to give the crude aldehyde which was purified by preparative HPLC using $PhCH_3$ as the eluting solvent to give pure 2-phenanthrenecarbaldehyde (5.1 g, 25%) mp 58—60° (lit. 59—59.5°, E. Mosettig and J. van dem Kamp, *J. Amer. Chem. Soc.* 55 2395 (1933)), (C, H).

23B. 2-Methyl-2-((2-phenanthrenylmethyl)amino)-1,3-propanediol hydrochloride

Using the reductive amination procedure outlined in 1A, 2-phenanthrene carbaldehyde and 2-amino-2-methyl-1,3-propanediol (Aldrich) gave 2-methyl-2-((2-phenanthrenylmethyl)amino)-1,3-propanediol hydrochloride mp 243—245°, (EtOH/$Et_2O$), (C, H, Cl, N).

Example 25

2-Acetoxymethyl-1,3-O-diacetyl-2-((1-pyrenylmethyl)amino)-1,3-propanediol

To a suspension of 2-hydroxymethyl-2-((1-pyrenylmethyl)amino)-1,3-propanediol hydrochloride (12, 2.50 g, 6.7 mmol) in 150 mL of dry THF was added 5 mL of acetylchloride (Aldrich, excess). The mixture was heated at reflux for 2 h, poured into 500 mL of cold 5% $NaHCO_3$ and then extracted with EtOAc (3 × 500 mL). The extacts were combined, washed with satd. NaCl solution, dried ($K_2CO_3$) and concentrated to 200 mL. This solution was boiled with a small amount of decolorizing charcoal and applied to a small plug (50 g) of $SiO_2$ Elution with 1L of EtOAc followed by concentration to 100 mL and dilution with 400 mL of hexane gave a crystalline product. This solid was collected by filtration and dried to give 2.73 g (88%) of 2-acetoxymethyl-1,3-O-diacetyl-2-((1-pyrenylmethyl)amino)-1,3-propanediol mp 150—150.5°, (C, H, N.).

Example 26

2-Ethyl-2-((1-pyrenylmethyl)amino)-1,3-propanediol diacetate

Using the procedure described for 25, 2-ethyl-2-((1-pyrenylmethyl)amino)-1,3-propanediol hydrochloride (6A) gave 2-ethyl-2-((1-pyrenylmethyl)amino)-1,3-propanediol diacetate mp 106—107°, (hexane), (C, H, N).

Example 27

2-Methyl-2-(((2,6,10-trichloro-9-anthracenyl)methyl)amino)-1,3-propanediol

27A. 2,6-Dichloroanthraquinone

Using the procedure of M. Nepras *et al.*, *Collection Czechoslov. Chem. Commun.* 28 2707 (1963), the disodium salt of anthraquinone-2,6-disulfonic acid (Aldrich) was converted to 2,6-dichloroanthraquinone mp 295—297° (lit. mp 291°, *Coll. Czech. Chem. Commun.* 28 2706 (1963)) (C, H, Cl).

27B, 2,6,10-Trichloro-9-anthracenecarbaldehyde

Using the procedure of V. I. Rogovik *et al.*, *Zh. Org. Khim.* 3 1315 (1967) except that the reaction was worked up after heating for 3.25 h at 95°, 2,6-dichloroanthraquinone (16.67 g, 0.06 mol.) gave 10.12 g of 2,6,10-trichloro-9-anthracenecarbaldehyde (54%) mp 269—271°, ($PhCH_3$), (C, H, Cl).

27C. 2-Methyl-2(((2,6,10-trichloro-9-anthracenyl)methyl)amino)-1,3-propanediol hydrochloride

Using the reductive amination procedure outlined in 1A, 2,6,10,-trichloroanthracene-9-carbaldehyde and 2-amino-2-methyl-1,3-propanediol (Aldrich) gave 2-methyl-2(((2,6,10-trichloro-9-anthrecenyl)methyl)-amino)-1,3-propanediol hydrochloride mp 275—277° (dec), (EtOH/$Et_2O$), (C, H, Cl N).

Example 28

2-(((6-Bromo-1-pyrenyl)methyl)amino)-2-methyl-1,3-propanediol and 2-((8-Bromo-1-pyrenylmethyl)amino)-2-methyl-1,3-propanediol

28A. 6 and 8-Bromopyrene-1-carbaldehyde

1-Bromopyrene was prepared by the procedure of W. H. Gumprecht, *Org. Syn. Coll. Vol.* V, 147 (1973), and formylated using the procedure outlined in 2A (except that $CH_2Cl_2$ was used as solvent with a reaction time of 2h), affording an approximately 1:1 ratio of 6- and 8-bromopyrene-1-carbaldehydes mp 168—181°, ($PhCH_3$/$CH_3OH$) (C, H, Br). This mixture was used without further purification.

28B. 2-((6-Bromo-1-pyrenylmethyl)amino)-2-methyl-1,3-propanediol hydrochloride and 2-((8-Bromo-1-pyrenylmethyl)amino)-2-methyl-1,3-propanediol hydrochloride

Using the reductive amination procedure described in 1A, the mixture of 6- and 8-bromopyrene-1-carbaldehydes (α1:1) and 2-amino-2-methyl-1,3-propanediol (Aldrich) gave a mixture of 2-((6-bromo-1-pyrenylmethyl)amino)-2-methyl-1,3-propanediol hydrochloride and 2-((8-bromo-1-pyrenylmethyl)amino)-2-methyl-1,3-propanediol hydrochloride (α1:1) mp 219—220°, (EtOH/$Et_2O$), (C, H, Br, Cl, N).

Example 29

2-Methyl-2-((1-pyrenylmethyl)amino)-1,3-propanediol diacetate

Using the procedure described for 25, 2-methyl-2-((1-pyrenylmethyl)amino)-1,3-propanediol hydrochloride (1A) gave 2-methyl-2-((1-pyrenylmethyl)amino)-1,3-propanediol diacetate mp 91—92°, (hexane), (C, H, N).

Example 30

2-Methyl-2-((4-pyrenylmethyl)amino)-1,3-propanediol

30A. 1,2,3,6,7,8-Hexahydro-4-pyrenecarbaldehyde

Using the formylation procedure outlined in 2A (except that $CH_2Cl_2$ was used as the solvent), 1,2,6,7,8 hexahydropyrene (Aldrich) gave 1,2,3,6,7,8-hexahydro-4-pyrenecarbaldehyde mp 102.5—105°, ($PhCH_3$/hexane), (C,H), (lit. mp 116°, J. P. Hoeffinger *et al., Bull. Soc. Chim. Fr.* 3099 (1969)).

30B. 4-Pyrenecarbaldehyde

1,2,3,6,7,8-Hexahydro-4-pyrenecarbaldehyde (11.81 g, 50 mmol) and 2,3-dichloro-5,6-dicyano-1,4-benzoquinone (Aldrich, 39.75 g, 0.175 mol) were refluxed for 4H in 1L of $PhCH_3$. The reaction mixture was cooled and then filtered. The filtrate was washed with 2N NaOH (3 × 500 mL), satd. NaCl solution (2 × 500 mL), dried ($NBa_2SO_4$), and passed through a plug of $SiO_2$(200 g). The solvent was then removed to give a bright yellow solid that was dried at 50° to give 8.91 g (77%) of 4-pyrenecarbaldehyde which was used without further purification. An analytical sample had mp 172—174°, ($CH_2Cl_2$/hexane), (C, H), (lit. mp 177—179°, Y. E. Gerasimenko and I. N. Shevebuk, *Zh. Org. Khim.* 4 2198 (1968)).

30C. 2-Methyl-2-((4-pyrenylmethyl)amino)-1,3-propanediol hydrochloride

Using the reductive amination procedure outlined in 1A, 4-pyrene-carbaldehyde (30B) and 2-amino-2-methyl-1,3-propanediol (Aldrich) gave 2-methyl-2-((4-pyrenylmethyl)amino)-1,3-propanediol hydrochloride·β$H_2O$ mp 225—227° (dec), (EtOH/$Et_2O$), (C, H, Cl, N).

Example 31

2-(((10-Butoxy-9-anthracenyl)methyl)amino)-2-methyl-1,3-propanediol

31A. 1-Butoxy-9-butylanthrone

31B. 9-Butoxyanthracene

31C. 10-Dibutyl-9,10-dihydro-9-anthrone

31D. 10-Butyl-9,10-dihydro-9-anthrone

A 2L 2-neck flask fitted with condenser, stirring bar, addition funnel and $N_2$ line was charged with anthrone (Aldrich, 100 g, 0.515 mol) and 500 mL of EtOH. To the mixture was added quickly a solution containing KOH (Mallinckrodt (85%), 35 g, 0.53 mol) dissolved in 250 ml of EtOH/$H_2O$ (5:1). The resulting deep red solution was warmed to 60°. BuBr (Fisher, 127.6 g, 0.93 mol) was added dropwise over 1 H to the reaction. The reaction was then stirred at 60° for 16 h. Most of the colour disappeared after 3H, leaving a deep yellow solution with precipitate (KBr). The mixture was cooled and filtered. The solvent was removed. The oily dark material was mixed with $PhCH_3$ (100 ml) and applied to a plug of $SiO_2$ (1 kg). Fractions of 250 mL were taken using $PhCH_3$ (5 L) as eluting solvent. Appropriate fractions were taken and further purified by preparative HPLC using $PhCH_3$ as eluting solvent and using normal and shave/recycle techniques. From the reaction the following materials were obtained (after separation and purification) in order of elution of $SiO_2$ with $PhCH_3$.

31A. 10-butoxy-9-butylanthracene mp 31—35°, 9.9 g (6%), (C, H), Rf = 0.83

31B. 9-Butoxyanthracene mp 86—87°, ($CH_3OH$), 52.11 g (44%), (C, H), Rf = 0.81

31C. 10-Dibutyl-9,10-dihydro-9-anthrone mp 108—109°, isolated after HPLC as an oil which solidified, 4.0 g (4%), (C, H), Rf = 0.53

31D. 10-Butyl-9,10-dihydro-9-anthrone isolated as an oil after HPLC, 41.2 g (29%), (C, H), Rf = 0.45 Anthrone (Rf = 0.35) was discarded upon isolation during the separations.

31E. 10-Butoxy-9-anthracenecarbaldehyde

10-Butoxyanthracene was formylated using DMF as both solvent and electrophile by the procedure of E. Campaigne and W. L. Archer, *J. Amer. Chem. Soc.* 75 989 (1953), affording 10-butoxy-9-anthracenecarbaldehyde mp 65—67°, (pentane), (C, H).

31F. 2-(((10-Butoxy-9-anthracenyl)methyl)amino)-2-methyl-1,3-propanediol hydrochloride

Using the reductive amination procedure outlined in 1A, 10-butoxy-9-anthracenecarbaldehyde and 2-amino-2-methyl-1,3-propanediol (Aldrich) gave 2-(((10-butoxy-9-anthracenyl)methyl)amino)-2-methyl-1,3-propanediol hydrochloride·mp 216—218° (dec), ($EtOH/Et_2O$), (C, H, Cl, N).

## Example 32

### 2-(((10-Butyl-9-anthracenyl)methyl)amino)-2-methyl-1,3-propanediol

32A. 10-Butyl-9-anthracenecarbaldehyde

10-Butyl-9,10-dihydro-9-anthrone (31D) was reduced by the procedure described by H. O. House *et al J. Org. Chem.* 38 1167 (1973) to give 9-butylanthracene (lit. mp 49°, A. Sieglitz and R. Marx, *Ber.* 56 1619 (1923)). This material was formylated by the procedure described in 2A (except that $CH_2Cl_2$ was used as the solvent) to give to 10-butyl-9-anthracenecarbaldehyde mp 79°, ($CH_2Cl_2$/pentane), (C, H), (lit. mp 80.5—81°, R. H. Martin, and L. van Hove, *Bull. Soc. Chim. Belg.* 61 504 (1952)).

32B. 2-(((10-Butyl-9-anthracenyl)methyl)amino)-2-methyl-1,3-propanediol hydrochloride

Using the reductive amination procedure outlined in 1A, 10-butyl-9-anthracenecarbaldehyde and 2-amino-2-methyl-1,3-propanediol (Aldrich) gave 2-(((10-butyl-9-anthracenyl)methyl)amino)-2-methyl-1,3-propanediol hydrochloride mp 225—227° (dec), ($EtOH/Et_2O$), (C, H, Cl, N).

## Example 33

### 2-((3-Fluoroanthenylmethyl)amino)-2-methylpropanol

33A. 3-Fluoranthenecarbaldehyde

33B. 7-Fluoranthenecarbaldehyde

33C. 8-Fluoranthenecarbaldehyde

33A. Formylation of Fluoranthene

Fluoranthene (Aldrich, 100 g, 0.49 mol) was formylated according to the prodedure outlined in 1A (except that $CH_2Cl_2$ was used as the reaction solvent). The crude material was passed through a plug of $SiO_2$ (1 kg) using $PhCH_3$ as the eluting solvent (3 L). The fractions containing the mixture of aldehydes were combined and the solvent removed giving 115 g of crude yellow oil. This material was dissolved in $CH_2Cl_2$ (500 mL) and diluted to 1 L with hexane. A yellow precipitate formed and was isolated by filtration. This solid (which is 3-fluoranthenecarbaldehyde) was crystallized from $CH_2Cl_2$/hexane and dried at 50° to give 45.7 g of pure material. The filtrate was added to the remaining impure mixture and the solvent removed. The remainder of the material was chromatographed on a plug of $SiO_2$ (1 kg) using $PhCH_3$ as eluting solvent and the fractions further purified by preparative HPLC, again using $PhCH_3$ as the eluting solvent. From this mixture, three aldehydes (including more of the 3 isomer) were obtained. The total amounts isolated, identity, and TLC behaviour ($SiO_2PhCH_3$) of these aldehydes are shown below.

33A. 3-Fluoranthenecarbaldehyde

68.73 g (61%) mp 103—104.5°, (Rf = 0.27), (C, H), (lit. mp 98—99°, N. Campbell and N. H. Wilson, *Chem. and Ind.* 1114 (1970).

33B. 7-Fluoranthenecarbaldehyde

2.10 g (2%) mp 139—141°, (C, H), Rf = 0.38

33C. 8-Fluoranthenecarbaldehyde

24.8 g (22%) mp 91.5—93°, (C, H), (Rf = 0.19).

33D: 2-((3-Fluoranthenylmethyl)amino)-2-methylpropanol hydrochloride

Using the reductive amination procedure outlined in 1A, 3-fluoranthenecarbaldehyde (33A) and 2-methyl-2-aminopropanol (Aldrich) gave 2-((3-fluoranthenylmethyl)-amino)-2-methyl-1-propanol hydrochloride, mp 288—290° (dec), ($CH_3OH/Et_2O$), (C, H, Cl, N).

Example 34

2-((8-Fluoranthenylmethyl)amino)-2-methyl-1,3-propanediol hydrochloride

Using the reductive amination procedure outlined in 1A, 8-fluoranthenecarbaldehyde (33C) and 2-methyl-2-amino-1,3-propanediol (Aldrich) gave 2-((8-fluoranthenylmethyl)amino)-2-methyl-1,3-propanediol hydrochloride mp 233—234.5° (dec), ($CH_3OH/Et_2O$), (C, H, Cl, N).

Example 35

2-(((3-Chloro-8-fluoranthenyl)methyl)amino)-2-methyl-1,3-propanediol and 2-(((4-Chloro-8-fluoranthenyl)methyl)amino)-2-methyl-1, 3-propanediol

35A. 3-Chlorofluoranthane

Fluoranthene (Aldrich, 99 g, 0.49 mol) dissolved in 2L of HOAc at 40° was treated dropwise with $SO_2Cl_2$ (Eastman, 47 mL, 0.59 mol), then heated at 100° for 1 h to obtain a mixture of dichloro and monochloro products plus starting material. The dichloro material (predominantly 1,3-dichlorofluoranthene, 16.8 g, 13%, mp 179—182°) precipitated out upon cooling overnight and was isolated by filtration. The filtrate was stripped to dryness and dissolved in hexane, then eluted through 500 g of $SiO_2$ with hexane to remove polar impurities. The hexane eluates were reduced to 200 mL to yield a precipitate that was recrystallized twice from $CH_2Cl_2$/hexane to afford 31.8 g of slightly impure 3-chlorofluoranthene (28%), mp 93—96°. A sample purified by preparative HPLC using hexane as the eluting solvent gave pure 3-chlorofluoranthene mp 95—98°, (lit. mp 96—98°, A. Sieglitz and H. Troster, *Berichte*, 96, 2577 (1963) (C, H, Cl).

35B. 3- and 4-Chlorofluoranthene-8-carboxyaldehyde

3-Chlorofluoranthene (35A, 30 g, 0.13 mol) was formylated according to the procedure outlined in 1A. Chromatography on a plug of $SiO_2$ (500 g) using $PhCH_3$ as the eluting solvent gave 14.2 g (42%) of purified material, a mixture of 3- and 4-chlorofluoranthene-8-carbaldehyde mp 165—183°, (C, H, Cl).

35C. 2-(((3-Chloro-8-fluoranthenyl)methyl)amino)-2-methyl-1,3-propanediol and 2-(((4-Chloro-8-fluorantheneyl)methyl)amino)-2-methyl-1,3-propanediol hydrochloride

Using the reduction amination procedure outlined in 1A the mixture of 3-and 4-chloro-fluoranthene-8-carbaldehyde and 2-amino-2-methyl-1,3-propanediol (Aldrich) gave a mixture of 2-(((3-chloro-8-fluoranthenyl)methyl)amino)-2-methyl-1,3-propanediol and 2-(((4-chloro-8-fluoranthenyl)methyl)amino)-2-methyl-1,3-propanediol hydrochloride, mp 224—225° (dec), ($EtOH/Et_2O$), (C, H, Cl, N).

Example 36

2-(((10-Ethoxy-9-phenanthrenyl)methyl)amino)-2-methyl-1,3-propanediol

36A 9-Ethoxy-1-phenanthrenecarbaldehyde

36B. 10-Ethoxy-9-phenanthrenecarbaldehyde

36C. 9-Ethoxy-3-phenanthrenecarbaldehyde

9-Ethoxyphenanthrene (Cambridge Chemical, Inc., 202 E. Smith St., Milwaukee, WI, 53207, 25 g, 0.113 mol) was formylated according to the procedure outlined in 2A, except that $CH_2Cl_2$ (500 mL) was used as the reaction solvent. Chromatography on a plug of $SiO_2$ (1 kg), followed by preparative HPLC using $PhCH_3$ as the eluting solvent yielded the three purified isomers described below:

36A. 9-Ethoxy-1-phenanthrenecarbaldehyde, 3.24 g (11%), (C, H), (Rf = 0.55, $SiO_2$, $PhCH_3$), mp 79—82°.

36B. 10-Ethoxy-9-phenanthrenecarbaldehyde, 15.0 g (60%), (C, H), (Rf = 0.50, $SiO_2$, $PhCH_3$), mo 67—70°.

36C. 9-Ethoxy-3-phenanthrenecarbaldehyde, 2.57 g (9%), (C, H), (Rf = 0.35, $SiO_2$, $PhCH_3$), mp 140—141°.

36D. 2-(((10-Ethoxy-9-phenanthrenyl)methyl)amino)-2-methyl-1,3-propanediol hydrochloride

Using the reductive amination procedure outlined in 1B, 10-ethoxy-9-phenanthraldehyde (36B) and 2-methyl-2-amino-1,3-propanediol (Aldrich) gave 2-(((10-ethoxy-9-phenanthrenyl)methyl)amino)-2-methyl-1,3-propanediol hyrochloride mp 199—200°, ($EtOH/Et_2O$), (C, H, Cl, N).

Example 37

2-(((9-Ethoxy-1-phenanthrenyl)methyl)amino)-2-methyl-1,3-propanediol

Using the reductive amination procedure outlined in 1A, 9-ethoxy-1-phenanthrenecarbaldehyde and 2-amino-2-methyl-1,3-propanediol (Aldrich) gave 2-(((9-ethoxy-1-phenanthrenyl)methyl)amino)-2-methyl-1,3-propanediol hydrochloride mp 215—216.5° (dec). ($EtOH/Et_2O$), (C, H, Cl, N).

Example 37

2-((3-Chrysenylmethyl)amino)-2-methyl-1,3-propanediol

37A. 2-Acetylchrysene

37B. 3-Acetylchrysene

37C. 6-Acetylchrysene

Friedel-Crafts reaction of chrysene with $AlCl_3/CH_3COCl/PhNO_2$ gave a mixture of 2-,3-, and 6-acetylchrysenes (285 g, Cambridge Chemical, Inc.). This was stirred and heated in $CH_2Cl_2$ (400 mL) and the filtered. The insoluble material, 2-acetylchrysene, was washed copiously with $CH_2Cl_2$. The filtrate was reduced to dryness and redissolved in $PhCH_3$, then chromatographed using $PhCH_3$ in portions over a plug of $SiO_2$ (1 kg) to separate the two remaining isomers. The faster eluting isomer is 6-acetylchrysene while the slower is 3-acetylchrysene. This yielded 132 g of impure 6-acetylchrysene, and 76 g of impure 3-acetylchrysene. A portion of the mixture was purified by preparative HPLC using $PhCH_3$ as the eluting solvent to yield:

37A. 2-Acetylchrysene mp 254—255°, (lit. mp 252—253°, W. Carruthers, *J. Chem. Soc.* 3486 (1953)) ($PhCH_3$), (C, H), (Rf = 0.50, $SiO_2$, $PhCH_3$);

37B. 3-Acetylchrysene mp 160—161°, (lit. mp 159°, W. Carruthers, *J. Chem Soc.* 3486 (1953)) ($CH_2Cl_2$/MeOH), (C, H), (Rf = 0.51, $SiO_2$, $PhCH_3$);

37C. 6-Acetylchrysene mp 141—142° (lit. mp 141°, W. Carruthers, *J. Chem Soc.* 3486 (1953)), ($CH_2Cl_2$/MeOH), (C, H), (Rf = 0.59, $SiO_2$, $PhCH_3$).

37D. Chrysene-3-carboxylic acid

3-Acetylchrysene (37D) was converted by the method of J. van de Kamp, *J. Amer. Chem. Soc.* 52, 3704 (1930) to chrysene-3-carboxylic acid mp 302—304° (dec), (lit. mp 295° (dec), W. Carruthers, *J. Chem Soc.* 3486 (1953)), (THF/EtOH), (C, H).

37E. Ethylchrysene-3-carboxylate

By the procedure of P. Arjunan and K. D. Berlin, *Org. Prep. and Procedures* 13 (5), 368 (1981), chrysene-3-carboxylic acid (37E, 18.3 g, 0.067 mol) was converted to ethyl chrysene-3-carboxylate 18.09 g (90%) mp 123—124°, ($PhCH_3$/hexane), (C, H).

37F. 3-Hydroxymethylchrysene

Ethyl chrysene-3-carboxylate (37E, 17.7 g, 0.059 mol) in 40 mL THF was treated with $LiBH_4$ (Morton Thiokol, Inc. — Alfa Products, PO Box 299, 152 Andover St., Danvers, MA 01923, 2.7 g, 0.12 mol) in three portions then refluxed for 3 days. The reaction mixture was poured into ice and acidified carefully to pH 1 with 1 M HCl. The precipitate was filtered and recrystallized from THF/hexane to afford 14.98 g (98%) of 3-chrysenemethanol mp 187—189°, (C, H).

37G. Chrysene-3-carbaldehyde

3-Hydroxymethylchrysene (37G, 14.6 g, 0.057 mol) in $CH_2Cl_2$ (2 L) was treated with $BaMnO_4$ (Aldrich, 29 g, 0.113 mol) and refluxed for 15 h. The reaction mixture was filtered, the solvent removed, and the resulting solid eluted through a plug of $SiO_2$ (500 g) using $PhCH_3$ as the eluting solvent. The appropriate fractions were combined and concentrated to 75 mL. Filtration and drying of the precipitate which formed gave 12.88 g (89%) of chrysene-3-carbaldehyde mp 177—177.5°, (C, H).

37H. 2-((3-Chrysenylmethyl)amino)-2-methyl-1,3-propanediol

Using the reductive amination procedure outlined in 18B, chrysene-3-carbaldehyde (37G) and 2-amino-2-methyl-1,3-propanediol (Aldrich) gave 2-((3-chrysenylmethyl)amino)-2-methyl-1,3-propanediol methanesulfonate mp 180—182° (dec), (EtOH/$Et_2O$), (C, H, N, S).

Example 38

2-((2-Chrysenylmethyl)amino)-2-methyl-1,3-propanediol

38A. Chrysene-2-carboxylic acid

Using the method of J. van de Kamp, *J. Amer. Chem. Soc.* 52 ,3704 (1930), except that the oxidation was run at 65° using pyridine as cosolvent to improve the solubility, 2-acetylchrysene (37A) was converted to chrysene-2-carboxylic acid mp 337—338° (lit. mp 325—327°, (dec) *J. Chem. Soc.* 3486 (1953)), (THF/EtOAc).

38B. Ethyl chrysene-2-carboxylate

By the procedure of P. Arjunan and K. D. Berlin, *Org. Prep. and Procedures* 13(5), 368 (1981), chrysene-2-carboxylic acid (38A, 10.4 g, 0.038 mol) was converted to ethyl chrysene-2-carboxylate 10.5 g (92%) mp 205—206°, ($PhCH_3$/hexane).

38C. 2-Hydroxymethylchrysene

Ethyl chrysene-2-carboxylate (38B, 10.1 g, 0.034 mol) in THF (500 mL) was treated with $LiBH_4$ (Alfa, 4×0.5 g 0.092 mol) under gentle reflux for 4 days. The reaction mixture was poured into ice and acidified carefully to pH 1 with 1M HCl. The precipitate was filtered and recrystallized from THF/hexane to afford 8.32 g (95%) of 2-chrysenemethanol mp 261—262°, (C, H).

38D. Chrysene-2-carbaldehyde

2-Hydroxymethylchrysene (38C 8.0 g, 0.031 mol) in $CH_2Cl_2$ (2.5 L) was treated with $BaMnO_4$ (Aldrich, 15.9 g, 0.062 mol) and refluxed for 12 H. The reaction mixture was filtered, and concentrated to give a solid, which was then passed through a plug of $SiO_2$ (500 g) using $PhCH_3$ as the eluting solvent. The appropriate fractions were combined and the volume reduced to 75 mL to afford 6.98 g (88%) of chrysene-2-carbaldehyde mp 215—216.5°, (C, H).

38E. 2-((2-Chrysenylmethyl)amino)-2-methyl-1,3-propanediol methanesulfonate

Using the reductive amination procedure outlined in 18C, chrysene-2-carbaldehyde (38D) and 2-amino-2-methyl-1,3-propanediol (Aldrich) gave 2-((2-chrysenylmethyl)amino)-2-methyl-1,3-propanediol methanesulfonate, mp 225—227° (dec), (abs. EtOH), (C, H, N, S).

Example 39

2-(((3-Ethyl-8-fluoranthenyl)methyl)amino)-2-methyl-1,3-propanediol and 1-(((4-Ethyl-8-fluoranthenyl)methyl)amino)-2-methyl-1,3-propanediol

39A. 3- and 4-Ethylfluoranthene-7-carbaldehydes

39B. 4-Ethylfluoranthene-3-carbaldehyde and 3-ethylfluoranthene-2-carbaldehyde

39C. 3- and 4-Ethylfluoranthene-8-carbaldehydes

3-Ethylfluoranthene (Cambridge Chemical, Inc., 70 g, 0.304 mol) was formylated according to the procedure outlined in 1A, except that $CH_2Cl_2$ (1 L) was used as the reaction solvent. Chromatography on a plug of $SiO_2$ (1 kg) yielded three partially purified products, each of which was rigorously purified by preparative HPLC using $PhCH_3$ as the eluting solvent. Each of the three products was an isomeric mixture as described below.

39A. 3- and 4-Ethylfluoranthene-7-carbaldehyde, 5.0 g (6%), (Rf = 0.55, $SiO_2$, $PhCH_3$), (C, H).

39B. 4-Ethylfluoranthene-3-carbaldehyde and 3-ethylfluoranthene-2-carbaldehyde, 4.7 g (6%), (Rf = 0.49, $SiO_2$, $PhCH_3$), (C, H).

39C. 3- and 4-Ethylfluoranthene-8-carbaldehyde, 47.3 g (60%), (Rf = 0.38, $SiO_2$, $PhCH_3$), (C, H).

39D. 4-Ethylfluoranthene-3-carbaldehyde

Product mixture 39B (4.7 g) was recrystallized twice from $CH_2Cl_2$/hexane to yield 1.83 g (2% from 3-ethylfluoranthene) of 4-ethylfluoranthene-3-carbaldehyde mp 113.5—116°, (C, H), (Rf = 0.31, $SiO_2$, $PhCH_3$).

39E. 2-(((3-Ethyl-8-fluoranthenyl)methyl)amino)-2-methyl-1,3-propanediol and 2-(((4-Ethyl-8-fluoranthenyl)methyl)amino)-2-methyl-1,3-propanediol hydrochloride

Using the reductive amination procedure outlined in 1A, the mixture of 3- and 4-ethyl-8-fluoranthenecarbaldehyde (39C) and 2-amino-2-methyl-1,3-propanediol (Aldrich) gave 2-(((3-ethyl-8-fluorthenyl)methyl)amino)-2-methyl-1,3-propanediol and 2-(((4-ethyl-8-fluoranthenyl) methyl)amino)-2-methyl-1,3-propanediol hydrochloride mp 182—183° (dec), (EtOH/$Et_2O$), (C, H, Cl, N).

Example 40

2-Methyl-2-((4-phenanthrenylmethyl)amino)-1,3-propanediol

40A. Phenanthrene-4-carbaldehyde

4-Phenanthrenemethanol (Aldrich, 29 g, 0.14 mol) was treated with pyridinium chlorochromate (PCC) (Aldrich, 45 g, 0.21 mol) in $CH_2Cl_2$ (2000 mL). After 6 h at RT, the reaction mixture was filtered through a plug of $SiO_2$ to give after removal of solvent and drying and crystallization from $CH_3OH$, 20.5 g (71%) of phenanthrene-4-carboxyaldehyde mp 82.5—84.5° (C, H).

40B. 2-Methyl-2-((4-phenanthrenylmethyl)amino)-1,3-propanediol hydrochloride

Using the reductive amination procedure outlined in 1A, phenanthrene-4-carbaldehyde and 2-amino-2-methyl-1,3-propanediol (Aldrich) gave 2-methyl-2-((4-phenanthrenylmethyl)amino)-1,3-propanediol hydrochloride mp 198—200° (dec), (EtOH/$Et_2O$), (C, H, Cl, N).

Example 41

2-Methyl-2-((3-phenanthrenylmethyl)amino)-1,3-propanediol

41A. Phenanthrene-3-carboxyaldehyde

Using the procedure outlined in 23A for 2-acetylphenanthrene, 3-acetylphenanthrene (Aldrich (technical grade), 25 g, 0.114 mol) gave 13G (51%) of phenanthrene-3-carboxylic acid. 15.6 g (.07 mol) of this acid was converted to the corresponding acid chloride, then to 8.37 g (58%) of phenanthrene-3-carboxaldehyde mp 78—80° (lit. 79.5—80°, E. Mosettig and J. van de Kamp, *J. Amer. Chem. Soc.* 55 2395 (1933)), (C, H).

41B 2-Methyl-2-((3-phenanthrenylmethyl)amino)-1,3-propanediol hydrochloride

Using the reductive amination outlined in 1A, phenanthrene-3-carbaldehyde and 2-amino-2-methyl-1,3-propanediol (Aldrich) gave 2-methyl-2-((3-phenanthrenylmethyl)amino)-1,3-propanediol hydrochloride mp 209—211°, ($EtOH/Et_2O$), (C, H, Cl, N).

Example 42

2-((2-Anthracenylmethyl)amino-2-methyl-1,3-propanediol

42A. (2-Anthracenyl)methanol

To a 2 L 3-neck flask fitted with condenser, thermometer and overhead stirrer was added 2-(hydroxymethyl)anthraquinone (Aldrich, 20 g, 0.084 mol), Zn dust (Mallinckrodt, 50 g, 0.765 mol), $CuSO_4{\cdot}5H_2O$ (Mallinckrodt, 0.5 g), and 28% $NH_4OH$ (Mallinckrodt, 600 mL). The temperature was increased to 80°, and the initial dark-red color faded (about 3 h). After refluxing for an additonal 30 min, the mixture was filtered. The filtrate was treated with conc. HCl until acidic, and the resulting precipitate collected. The zinc solid was extracted with EtOAc until the washings were clear, and the EtOAc removed to give an oil. The oil and the precipitate were added to a mixture of conc. HCl (12 ml) in *i*-PrOH (1200 mL). This solution was concentrated by rotary evaporation to near dryness and $CH_3OH$ (100 mL) was added to give after filtration and drying 13.48 g (77%) of (2-anthracenyl)methanol mp 218—221° (lit mp 223—224°, P. Arjunan and K. D. Berlin, *Org. Prep. and Procedure.*, 13(5), 368 (1981)).

42B. 2-Anthracenecarbaldehyde

2-Anthracenylmethanol (1.0 g, 0.0048 mol) in $CH_2Cl_2$ (700 mL) was treated with pyridinium chlorochromate (PCC) (Aldrich, 21.56 g, 0.1 mol) and heated for 4 h. The reaction was cooled and filtered through a plug of $SiO_2$ (500 g) using $CH_2Cl_2$ as the eluting solvent. After removal of the solvent the crude material was chromatographed on $SiO_2$ with $PhCH_3$ as the eluting solvent to afford 0.5 g (51%) of 2-anthracenecarbaldehyde mp 201—202.5° (lit mp 202—203°, P. H. Gore, *J. Chem. Soc.* 1616 (1959)), (C, H).

42C. 2-((2-Anthracenylmethyl)amino-2-methyl-1,3-propanediol hydrochloride 9/20 $H_2O$

Using the reductive amination procedure outlined in 1A, 2-anthracenecarbaldehyde and 2-methyl-2-amino-1,3-propanediol (Aldrich) gave 2-((2-anthracenylmethyl)amino-2-methyl-1,3-propanediol hydrochloride·9/20 $H_2O$ mp 224—226°, ($EtOH,Et_2O$), (C, H, Cl, N).

.Example 43

(+−)(2R*,3R*)-2-((1-Pyrenylmethylamino)-2-methyl-1,3-butanediol

43A. (+−)(2R*,3S)-2-Methyl-2-nitro-1,3-butanediol and

43B. (+−)(2R*,3S*)-2-Methyl-2-nitro-1,3-butanediol

To a mixture of 2-nitro-1-propanol (Aldrich, 63.0 g, 0.60 mol) and acetaldehyde (Eastman, 39.6 g, 0.90 mol) cooled in an ice bath under $N_2$ was added cold $H_2O$ (40 mL) and calcium hydroxide (200 mg). The mixture was allowed to warm to RT over 2 h and then stirred for 68 h. The resulting solution was neutralized with excess solid $CO_2$. The mixture was stirred for 1 h before filtration through a Millipore filter (Millipore Corp., Bedford, MA, 01730). The filtrate was then concentrated under vacuum at 35°. The residue, a viscous syrup which partially crystallized on drying under vacuum, (0.1 mm, RT, 48 h), was triturated with cold $Et_2O$ (35 mL). Solid white crystals which formed were collected by filtration, washed with cold $Et_2O$ (3 × 15 mL) and dried under vacuum (0.1 mm, RT) to give 34.1 g of material judged by NMR to be diastereomer 43A (purity ]97%, racemic). After recrystallization, the diasteroiomeric purity was ]99%, mp 78.5—81° (lit. 78°; cf. *Beil* 1, 482 in Henry, *Bull. Soc. Chim. Fr.* β3₵ 15, 1224), (C, H, N).

The original filtrate (including wash) was concentrated under vacuum to a pale yellow liquid which was subjected to flash chromatography as follows: The sample was mixed with hexane: EtOAc (2:1, 100 mL) and added to a column of dry $SiO_2$ (1.5 kg). The column was eluted with hexane: EtOAc (2:1, 12 L) the hexane:EtOAc (1:1, 6 L) while 500 mL fractions were collected. Appropriate fractions were combined. Pure product was found in the final 8 L; yield 38.7 g of viscous syrup, judged by NMR to be a 1:1 mixture of the two racemic diastereomers (43A and 43B), (C, H, N).

This and another batch of the 1:1 diasteriomeric mixture (prepared as described above) were combined (67 g, total) and subjected to successive liquid-liquid partitioning between $H_2O$ and EtOAc to give pure samples (α99% on the basis of NMR and HPLC (Hamilton PRP—1 column using 3.5% aqueous

acetonitrile as the mobile phase)) of 43A (24.9 g, k'=4.3, C, H, N) and 43B (15.8 g, k'=2.1, C, H, N, a colorless, viscous liquid).

43C. (+−)(2R*,4S*,5R*)-4,5-dimethyl-5-nitro-2-phenyl-1,3-dioxane and

43D. (+−)(2R*,4S*,5R*)-4,5-dimethyl-5-nitro-2-phenyl-1,3-dioxane

The relative configurations of these two diasteriomeric pairs (43A and 43B) were assigned on the basis of comparative NMR analysis of the respective cyclic acetals derived from benzaldehyde. Thus, 43A (1.49 g, 0.01 mol) and benzaldehyde (Mallinckrodt, 1.06 g, 0.01 mol) were condensed in benzene in the presence of a catalytic amount of *p*-toluenesulfonic acid with azeotropic removal of water (according to the method of H. Piotrowska, B. Serafin and T. Urbanski, *Tetrahedron* 109, 379 (1963)). After successive washing with satd. $NaHCO_3$ solution, drying ($MgSO_4$), filtration, and removal of the benzene by rotary evaporation, a pale yellow solid was obtained. A solution of this product in ethanol at 0°C provided an oil which was isolated by decanting the mother liquor and drying under vacuum (0.1 mm, RT). The yield was 1.48 g (62%) of (+−)(2*R**,4*S**,5*R**)-4,5-dimethyl-5-nitro-2-phenyl-1,3-dioxane (C, H, N).
Similarly prepared from 43B and benzaldehyde was (+−)(2*R**,4*S**,5*S**)-4,5-dimethyl-5-nitro-2-phenyl-1,3-dioxane (74%) (C, H, N).

43E. (+−)(2R*,3R*)-2-Amino-2-methyl-1,3-butanediol acetate

To a solution of (+−)(2*R**,3*R**)-2-methyl-2-nitro-1,3-butanediol (43B, 22.1, 0.148 mol) 95% EtOH (150 mL) was added glacial acetic acid (25 mL) and 10% Pd/C (MCB, 2.0 g). The reduction was carried out in a Parr apparatus at 50 psi of $H_2$ during a 48 h period at RT. The catalyst was removed by filtration through a Millipore- filter, and the solvent removed under vacuum (2 days). The viscous, colorless syrup wa dissolved in abs. EtOH (30 mL). Dilution with abs. $Et_2O$ (300 mL) gave a cloudy liquid which was placed in a refrigerator for two days. During this time, colorless crystals formed. They were washed with $Et_2O$ and dried in a vacuum oven at RT for two days. The yield of (+−)(2*R**,3*R**)-2-amino-methyl-1,3-butanol acetate was 25.6 g (97%) mp 117—121°, (C, H, N).

43F. (+−)(2*R**,3*R**)-2-Amino-2-methyl-1,3-butanediol acetate

Prepared from (+−)(2*R**,3*R**)-2-methyl-2-nitro-1,3-butanediol (43A) as described for 43E (93%) (C, H, N) mp 163—165°C.

43G. (+−)(2*R**,3*S**)-2-((1-Pyrenylmethyl)amino)-2-methyl-1,3-butanediol

To a RB flask was added (+−)(*R**,*S**)-2-amino-2-methyl-1,3-butanediol acetate (43F) and an equimolar amount of sodium methoxide (MCB) and $CH_3OH$ (100 mL). After warming the solvent was removed by rotary evaporation and after addition of 1-pyrenecarbaldehyde (Aldrich) the reaction run following the normal reductive amination procedure outlined in 1A to give (+−)(2*R**,3*S**)-2-((1-pyrenylmethyl)amino)-2-methyl-1,3-butanediol hydrochloride mp 221—222° (dec), ($EtOH/Et_2O$), (C, H, Cl, N)

Example 44

(+−)(2R*,3S*)-2-((9-Phenanthrenylmethyl)amino)-2-methyl-1,3-butanediol hydrochloride

Using the procedure outlined for 43G, phenanthrene-9-carbaldehyde (Aldrich) and (+−)(3*R**,3*S**)-2-amino-2-methyl-1,3-butanediol acetate (43F) gave (+−)(2*R**,3*S**)-2-((9-phenanthrenylmethyl)amino)-2-methyl-1,3-butanediol hydrochloride mp 204—206° (dec), ($EtOH/Et_2O$), (C, H, Cl, N)

Example 45

2-Ethoxymethyl-2-((1-pyrenylmethyl)amino)-1,3-propanediol

45A. 3,5-Diphenyl-7a(7H)-ethoxymethyl-1H,3H,5H-oxazolo(3,4-c)oxazole

A mechanically stirred 60% dispersion of NaH in mineral oil (Alfa, 34.0 g, 0.85 mol) was washed with dry hexane to remove the oil and suspended in dry DMF (300 mL). To the mixture was added a solution of 3,5-diphenyl *1H,3H,5H*-oxazolo(3,4-*c*)oxazole-7a(7*H*)-methanol (208.2 g, 0.7 mol, prepared by the method of J. Pierce *et., J. Amer. Chem. Soc.* 73 2595 (1951)) in dry DMF (300 mL) keeping the reaction mixture between 30—35°. The salt suspension was stirred at RT for 60 min, diluted with dry DMF (200 mL) to facilitate stirring, cooled, then treated with ethyl iodide (Aldrich, excess) at such a rate that the reaction temperature was between 20—35°. The mixture was stirred at RT for 2 h, then cautiously treated with absolute EtOH (30 mL). The resulting mixture was diluted with $Et_2O$ (2.5 L) and the resulting solids removed by filtration. The solvent was then removed using a rotary evaporator to give 229.5 g of a yellow oil containing both starting material and desired product. A solution of the oil in chloroform was mixed with $SiO_2$ (200 g) and the solvent removed. The solid was then added to a column of $SiO_2$ (800 g). Elution with the EtOAc/hexane (1:3.5) gave 139.7 g (61.3%) of 3,5-diphenyl-7a(7*H*)-ethoxymethyl-1*H*,3*H*,5*H*-oxazolo(3,4-*c*)oxazole. An analytical sample was obtained by recrystallization from hexane, mp 83.5—85°, (C, H, N). The bulk of the material was used without further purification.

B. 2-Amino-2-ethoxymethyl-1,3-propanediol hydrochloride β $H_2O$

3,5-Diphenyl-7a(7*H*)-ethoxymethyl-1*H*,3*H*,5*H*-oxazolo(3,4-*c*)oxazole (136 g, 0.42 mol) was dissolved in 6N HCl (400 mL) and the resulting solution stirred 1.5 h at RT. After extraction with $Et_2O$ (2×200 mL) to

remove benzaldehyde, the aqueous solution was concentrated on a rotary evaporator to give a colorless oil. This was cooled in an ice bath to facilitate crystallization. The solid which formed was slurried with cold $CH_3CN$, filtered, then washed with $Et_2O$ and dried in a vacuum oven at RT to give 71 g (89%) of 2-amino-2-ethoxymethyl-1,3-propanediol hydrochloride·β $H_2O$ mp 78—79°, (C, H, Cl, N).

45C. 2-Ethoxymethyl-2-((1-pyrenylmethyl)amino)-1,3-propanediol hydrochloride $\frac{1}{3}$ $H_2O$

To a RB flask was added 2-amino-2-ethoxymethyl-1,3-propanediol hydrochloride·β $H_2O$ (45B) and an equimolar amount of sodium methoxide (MCB) and $CH_3OH$ (100 mL). After warming the solvent was removed by rotary evaporation and after addition of pyrene-1-carbaldehyde (Aldrich) the reaction run following the normal reductive amination procedure outlined in 18C to give 2-ethoxymethyl-2-(((1-pyrenyl)methyl)amino)-1,3-propanediol hydrochloride·$\frac{1}{3}H_2O$, mp 189—190° (dec), ($EtOH/Et_2O$), (C, H, Cl, N).

Example 46

2-Ethoxymethyl-2-((9-phenanthrenylmethyl)amino)-1,3-propanediol hydrochloride

Using the reductive amination procedure outlined for 45C, phenanthrene-9-carbaldehyde (Aldrich) and 2-amino-2-ethoxymethyl-1,3-propanediol hydrochloride·β$H_2O$ (45B) gave 2-ethoxymethyl-2-(((9-phenanthrenyl)methyl)amino)-1,3-propanediol hydrochloride mp 173—175°, ($EtOH/Et_2O$), (C, H, Cl, N).

Example 47

2-((6-Chrysenylmethyl)-N-methylamino)-2-methyl-1,3-propanediol

47A. 6-Hydroxymethylchrysene

A solution of chrysene-6-carboxaldehyde (2A, 25 g, 0.098 mol) in THF (500 mL) was treated with $NaBH_4$ (Aldrich, 1.85 g, 0.049 mol) in portions with ice cooling. After stirring at RT overnight, the reaction mixture was poured into ice water and acidified carefully to pH 1 with 0.1 M HCl. The precipitate was filtered and recrystallized from THF/hexane to afford 23.4 g (93%) of 6-chrysenemethanol mp 206—207° (lit mp 204—206°), (C,H).

47B. 6-Chloromethylchrysene

A suspension of 6-hydroxymethylchrysene (47A, 10.7 g, 0.041 mol) in $PhCH_3$ (700 mL) was treated with $SOCl_2$ (Eastman, 10 mL, 0.139 mol). The mixture was warmed to 70° for 12 h, then vigorously refluxed for 1 h to drive off HCl and $SO_2$ gases. The reaction was cooled and the solvent removed by rotary evaporation to give a crude solid. This was redissolved in $PhCH_3$ and the solvent removed again. The crude 6-chloromethylchrysene was used immediately without further purification.

47C. 2,2,5-Trimethyl-5-amino-1,3-dioxolane

A magnetically stirred mixture of 2-amino-2-methyl-1,3-propanediol (Aldrich, 105.14 g 1.00 mol), *p*-toluenesulfonic acid monohydrate (Fisher, 192.12 g 1.01 mol), and $PhCH_3$ (300 mL) was refluxed with removal of $H_2O$ until the theoretical amount of $H_2O$ was obtained), then cooled. The supernatant $PhCH_3$ layer was decanted and the redisual solid dissolved in DMF (400 mL) containing 2,2-dimethoxypropane (Aldrich, 300 mL) and *p*-toluenesulfonic acid monohydrate (3.0 g, 0.16 mol). The solution was refluxed for 48 h. The solvents were removed by distillation until the pot temperature reached 120°. This process was repeated twice using additional 2,2-dimethoxypropane (350 mL) for 15 h reflux, then with 2,2-dimethoxypropane (400 mL) for 6 h. The cooled residue was diluted with $Et_2O$ (5 L) and the salt which precipitated collected by filtration. The solid was treated with 25% aqueous NaOH (300 mL), saturated with NaCl, and the resulting free base was extracted into $Et_2O$ (3×600 mL). The combined extracts were dried over $Na_2SO_4$, then fractionally distilled to give 38.30 g (26.4%) of 2,2,5-trimethyl-5-amino-1,3-dioxolane as a colorless liquid bp 64—67°/15 mm. The synthesis of this compound is reported in FR 1,457,767 (CA *68*:49617 z) using acetone as the ketalization agent but no physical constants were given.

47D. N,2,2,5-Tetramethyl-1,3-dioxolan-5-amine

A solution of *N*-(2,2,5-Trimethyl-1,3-dioxolan-5-yl)formamide (bp 103—106° prepared from 2,2,5-trimethyl-5-amino-1,3-dioxolane by the procedure in FR 1,457,767 (CA *68*:49617z)) (83.0 g, 0.479 mol) in dry THF (200 mL) was added dropwise to a mechanically stirred solution of lithium aluminum hydride (Alfa, 22.76 g, 0.70 mol) in dry THF (900 mL) at such a rate that the internal temperature did not rise above 55°. The resulting mixture was then cautiously heated to reflux (a vigorous exotherm occurred, then quickly subsided), then refluxed for 5 h and let stir overnight. The resulting mixture was cooled with an ice bath, then cautiously treated successively with $H_2O$ (23 mL), 15% NaOH (23 mL), then $H_2O$ (23 mL). The heterogeneous mixture was filtered and the solid washed with THF (200 mL). The combined organic solutions were fractionally distilled to give 62.50 g (82%) of *N*,2,2,5-tetramethyl-1,3-dioxolan-5-amine as an oil bp 63—64°/12 mm, (C, H, N).

47E. 2-((6-Chrysenylmethyl)-N-methylamino)-2-methyl-1,3-propanediol methanesulfo-nate

In a RB flask was placed 6-chloromethylchrysene (47B, 10.0 g, 36 mmol), *N*,2,2,5-tetramethyl-1,3-diox-olan-5-amine (47D, 7.96 g, 50.0 mmol), $K_2CO_3$ (Mallinckrodt, 13.8 g, 0.1 mol) and abs. EtOH (500 mL). The mixture was refluxed overnight under $N_2$. The mixture was cooled, the solvent removed and the solid

washed with $H_2O$ (3×500 mL) to remove inorganic material. The solid was then refluxed for 1 h in 10% HCl (500 mL). The mixture was then cooled, basified with 5N NaOH (200 mL) and filtered to give a white solid. This was washed with $H_2O$ (3×500 mL) and sucked dry. The solid was dissolved in a solution of $CH_3SO_3H$ (Alfa, (99.5%), 4 mL) in abs EtOH (300 mL) and filtered through a sintered glass funnel. The filtrate was then diluted to 4L with $Et_2O$. The solid was filtered and further recrystallized from abs. EtOH/$Et_2O$ 3X to give 7.18 g (43.8%) of 2-((6-chrysenylethyl)-*N*-methylamino)-2-methyl-1,3-propanediol methanesulfonate mp 172—173° (dec), (EtOH/$Et_2O$), (C, H, N, S).

## Example 48

### 2-(N-(3-Fluoranthenylmethyl)methylamino)-2-methyl-1,3-propanediol

48A. 3-Hydroxymethylfluoranthene

A solution of 3-fluoranthenecarbaldehyde (33A, 20 g, 0.087 mol) in THF (200 mL) was treated with $NaBH_4$ (MCB Manufacturing Chemists, Inc., 2909 Highland Ave., Cincinnatti, OH, 45212, 1.64 g, 0.043 mol) in portions. After stirring overnight, the reaction mixture was poured into ice water and acidified to pH 3 with 0.1 M HCl. The precipitate was filtered and recrystallized from $CH_2Cl_2$/hexane to afford 16.2 g (80%) of 3-fluoranthenemethanol mp 137—138°. *Vopr. Khim. Khim. Teknol.*, 51, 60 (1978) (Russ.) (not in CA) mentions the compound, but no mp is given (C, H).

48B. 3-Chloromethylfluoranthene

To a suspension of 3-hydroxymethylfluoranthene (48, 8.2 g 0.035 mol) in $PhCH_3$ (500 mL) was added $SOCl_2$ (Eastman 6.5 mL, 0.090 mol). The mixture was warmed at 70° for 12 h, then vigorously refluxed for 1 h to drive off HCl and $SO_2$ gas. The reaction was cooled and the solvent removed by rotary evaporation to give a crude solid. This was redissolved in $PhCH_3$ and the solvent removed again. The crude 3-chloromethylfluoranthene was used immediately without further purification.

48C. 2-(N-3-Fluoranthenylmethyl)methylamino)-2-methyl-1,3-propanediol methanesulfonate

Using the procedure for 47E, 3-chloromethylfluoranthene (48B) and *N*,2,2,5-tetramethyl-1,3-dioxolan-5-amine (47D) gave 2-(*N*-3-fluoranthenylmethyl)methylamino)-2-methyl-1,3-propanediol methanesulfonate, mp 138—140° (dec), (EtOH/$Et_2O$), (C, H, N, S)

## Example 49

### 3-Methoxy-2-methyl-2-((1-pyrenylmethyl)amino)-1-propanol

49A. 4-Aza-3-hydroxymethyl-3-methyl-1-oxaspiro[4.5]decane

A solution of 2-amino-2-methyl-1,3-propanediol (Aldrich, 303.4 g, 3.0 mol), cyclohexanone (Fisher, 294.5 g, 3.0 mol) and $PhCH_3$ (400 mL) was refluxed for approximately 2 h with azeotropic removal of $H_2O$. The material which crystallized from the $PhCH_3$ on cooling was recrystallized twice from hexane to give 444.4 g of 4-aza-3-hydroxymethyl-3-methyl-1-oxaspiro β 4.5 ¢ decane (80%) mp 52—54°, (C, H, N).

49B. 4-Aza-3-methoxymethyl-3-methyl-1-oxaspiro[4.5]decane

A mechanically stirred 60% dispersion of NaH in mineral oil (Alfa, 75 g, 1.9 mol) was washed with dry hexane to remove the oil and suspended in dry DMF (200 mL). To the mixture was added a solution of 4-aza-3-hydroxymethyl-3-methyl-1-oxaspiro[4.5]decane (27.8 g, 1.5 mol) in dry DMF (200 mL) keeping the reaction mixture temperature between 30—35°. Small amounts of DMF were added as necessary to facilitate stirring. The mixture was stirred at RT for 1.5 h, then cooled and treated with methyliodide (Fisher, 234.2 g, 102.7 mL, 1.65 mol) keeping the reaction temperature between 20—30°. The mixture was stirred 2 h at RT and slowly treated with absolute EtOH (40 mL), then diluted with dry $Et_2O$ (3 L). The reaction mixture was filtered and the solvent removed by rotary evaporation. The residue was then fractionally distilled to give 209.7 g (70.3%) of 4-aza-3-methoxymethyl-3-methyl-1-oxaspiro[4.5]decane as a colourless liquid bp 114°/14 mm, (C, H, N).

49C. 2-Amino-3-methoxy-2-methyl-1-propanol

A solution of 4-aza-3-methoxymethyl-3-methyl-1-oxaspiro(4.5)decane (299 g, 1.5 mol) and 6 N HCl (500 mL) was refluxed for 60 min. On cooling, two layers formed, the upper one containing cyclohexanone was removed by extraction with $Et_2O$ (2×400 mL). The lower aqueous layer was concentrated on a rotary evaporator to give a syrup which then was treated with excess 50% NaOH. The resulting slurry was extracted with $Et_2O/CH_2Cl_2$ (2:1, 4×500 mL), then with $CH_2Cl_2$ (500 mL). The solvent was removed by rotary evaporation to give 198 g of pale oil. Fractional distillation of this oil gave 166 g (93%) of 2-amino-3-methoxymethyl-1-propanol as a colorless oil, bp 94°C/17 mm, (C, H, N).

49D. 3-Methoxy-2-methyl-2-((7-pyrenylmethyl)amino)-1-propanol methanesulfonate $\frac{1}{3}$ $H_2O$

Using the reductive amination procedure outlined in 18C, pyrene-1-carbaldehyde (Aldrich) and 2-amino-3-methoxy-2-methyl-1-propanol (49C) gave 3-methoxy-2-methyl-2-((1-pyrenylmethyl)amino)-1-propanol methanesulfonate·$\frac{1}{3}H_2O$ mp 158—160°, (EtOH/$Et_2O$), (C, H, N, S).

Example 50

3-Methoxy-2-methyl-2-((9-phenanthrenylmethyl)amino)-1-propanol hydrochloride

Using the reductive amination procedure outlined in 18C, phenanthrene-9-carbaldehyde (Aldrich) and 2-amino-3-methoxy-2-methyl-1-propanol (49C) gave 3-methoxy-2-methyl-2-((9-phenanthrenylmethyl)amino)-1-propanol hydrochloride mp 211—213° (dec), ($EtOH/Et_2O$), (C,H,Cl,N).

Example 51

2-((3-Perylenylmethyl)amino)-3-methoxy-2-methyl-1-propanol methanesulfonate 1/4$H_2O$ 3/10 EtOH

Using the reductive amination procedure outlined in 1B, perylene-3-carbaldehyde (18A) and 2-amino-3-methoxy-2-methyl-1-propanol (49C) gave 2-((3-perylenylmethyl)amino)-3-methoxy-2-methyl-1-propanol methanesulfonate·1/4$H_2O$·3/10 EtOH 194—197° (dec), ($EtOH/Et_2O$), (C, H, N, S)

Example 52

(+−)(2R*,3S*)-2-((3-Perylenylmethyl)amino)-2-methyl-1,3-butanediol methanesulfonate 2/10 $H_2O$

Using the reductive amination procedure described for 43G, perylene-3-carbaldehyde and (+−)(2*R**,3*S**)-2-amino-2-methyl-1,3-butanediol acetate (43F) gave (+−)(2*R**,3*S**)-2-((3-perylenylmethyl)amino)-2-methyl-1,3-butanediol methanesulfonate·2/10 $H_2O$ mp 193.5—194.5° (dec), (C, H, N, S).

Example 53

2-((3-Perylenylmethyl)amino-2-ethoxymethyl-1,3-propanediol methanesulfonate 3/4 $H_2O$

Using the reductive amination procedure outlined for 45C, perylene-3-carbaldehyde (18A) and 2-amino-2-ethoxymethyl-1,3-propanediol hydrochloride 1/4 $H_2O$ (45B) gave 2-((3-perylenylmethyl)amino)-2-ethoxymethyl-1,3-propanediol methanesulfonate·3/4$H_2O$ mp 188—190° (dec), ($EtOH/Et_2O$), (C, H, N, S).

Example 54

2-Methyl-1-((9-Phenanthrenylmethyl)amino)-1,4-butanediol

54A. Ethyl *N*-benzylidene-*ℓ*-alaninate

Ethyl *N*-benzylidene-*ℓ*-alaninate was prepared according to the general procedure of G. Stork *et al., J. Org. Chem.* 41, 349 (1976) bp 98—100°/0.4 mm (lit. 100°/0.3 mm, A. Calcagni *et al.,* Synthesis 455 (1981)).

54B. 2-(2-Iodoethoxy)tetrahydro-2-H-pyran

Freshly distilled dihydropyran (Aldrich, 59.0 g, 0.7 mol) was added dropwise to a cooled solution of idoethanol (Aldrich, 98 g, 0.57 mol) in $Et_2O$ (1 L) containing 0.1 g of *p*-toluenesulfonic acid (Eastman). The solution was then stirred for 1 h at 5°. Solid $K_2CO_3$ (Mallinckrodt, 5 g) was then added to the reaction mixture and the resulting suspension stirred an additional 1 h at RT. The reaction was then filtered and the remaining solid washed with $Et_2O$ (1 L). The organic solutions were combined and concentrated on a rotary evaporator (in a flask washed with 1% $NEt_3$ in $H_2O$). The crude 2-(2-iodoethoxy)-tetrahydro-2-*H*-pyran (α100 g, 68.9%) was used without further purification.

54C. Ethyl 2-benzylideneamino-2-methyl-4-((tetrahydro-2-H-pyran-2-yl)oxy)butyrate

A solution of lithium diisopropylamide was prepared by dropwise addition of *n*-BuLi (Aldrich, 1.6 M in hexane, 228 mL, 0.365 mol) to a solution of diisopropylamine (Aldrich, 51.6 g, 0.51 mol) in a mixture of dry THF (700 mL) and dry HMPA (Aldrich, 40 mL) kept at 30—40°. The solution was then cooled to −70° and a solution of ethyl *N*-benzylidene-*L*-alaninate (54A, 74.9 g, 0.365 mol) was added dropwise to the solution allowing the reaction mixture warm to −20° for several min. The resulting red solution was then cooled to −70°. (2-Iodoethoxy)tetrahydro-2-H-pyran (54B, 98.1 g, 0.383 mol) was then added to the solution at such a rate that the temperature in the reaction mixture did not rise above −65°. The solution was allowed to warm slowly to RT and stirred for 14 h. The volume of the solution was reduced to α300 mL by a stream of dry $N_2$ during the last few hours to facilitate the final workup. The reaction was quenched with sat. NaCl (800 mL) and diluted with $Et_2O$ (800 mL). The $Et_2O$ was removed and the aqueous layer extracted with hexane (500 mL). The $Et_2O$ and hexane layers were combined and dried ($Na_2SO_4$). The solution was filterd and the solvent removed to give 124 g of crude red oil. Bulb to bulb distillation (in 1% aq. $NEt_3$ washed glassware) (210° bath temperature/0.3 mm) gave 95 g of ethyl e-benzylideneamino-2-methyl-4-((tetrahydero-2*H*-pyran-2-yl)oxy)butyrate which was homogeneous by vpc and gave acceptable NMR and mass spectra. It was stored under $N_2$ in the refrigerator and was used without further purification.

54D. Ethyl 2-benzylideneamino-2-methyl-4-((tetrahydro-2H-pyran-2-yl)oxy)butyrate

A solution of ethyl 2-benzylideneamino-2-methyl-4-((tetrahydro-2*H*-pyran-2-yl)oxy)butyrate (54C, 100.0 g, 0.3 mol) in THF (100 mL) was added slowly to a suspension of lithium aluminum hydride (Alfa, 22.77 g, 0.6 mol) rapidly stirred in dry THF (1 L) at such a rate to maintain a gentle reflux. After the addition was complete the mixture was refluxed for 4 h. The reaction mixture was cooled and treated successively with $H_2O$ (23 mL), 15N NaOH (23 mL) and $H_2O$ (45 mL). The solid was removed by filtration and washed with THF (200 mL). The organic layers were combined and concentrated by rotary evaporation to give ethyl

2-benzylideneamino-2-methyl-4-((tetrahydro-2*H*-pryan-2-yl)oxy)butyrate (81.1 g, 92.0%) as a thick oil which was used without further purification,

54E. 2-Benzylamino-2-methyl-1,4-butanediol

The crude ethyl 2-benzylideneamino-2-methyl-4-((tetrahydro-2*H*-pyran-2-yl)oxy)butyrate (54D, 80.1 g, 0.273 mol) was dissolved in 3N HCl (128 mL). After 5 min the mixture was washed with $Et_2O$ (200 mL). The aqueous solution was concentrated by rotary evaporation to give a thick oil which was cooled and basified with excess 50% NaOH. The oily amine which formned was extracted with $Et_2O$ (3 × 200 mL). The $Et_2O$ extracts were combined and concentrated to give 63.6 g of a thick oil. Distillation gave 49.8 g (94%) of 2-benzylamino-2-methyl-1,4-butanediol as a pale yellow oil (bp 168—170°/0.35 mm), (C, H, N).

54F. 2-Amino-2-methyl-1,4-butanediol hydrochloride

2-Benzylamino-2-methyl-1,4-butanediol (54E, 31.08 g, 0.149 mol) was dissolved in 95% EtOH (240 mL) containing concentrated HCl (21 mL, 0.25 mol) and 5% Pd/C (10.0 g) and reduced in a Parr apparatus at 40 psi over 37 h at RT. The catalyst was then removed by filtration and the solvent removed by rotary evaporation (bath at 60°) to give 20.91 g of 2-amino-2-methyl-1,4-butanediol hydrochloride (90.2%) as a clear, thick, colourless oil with acceptable NMR and mass spectra. It was used without further purification. This compound has been reported as its acetate salt (G. Cardillo *et al.,* Chem. Commun. 1308, 1982), but no date was given. Attempts to duplicate the latter procedure we're unsuccessful.

54G. 2-Methyl-2-((9-phenanthrenylmethyl)amino)-1,4-butanediol methanesulfonate 3/10 $H_2O$

To a RB flask was added 2-amino-2-methyl-1,4-butanediol hydrochloride (54F) and an equal amount of sodium methoxide (MCB) and enough $CH_3OH$ to form a solution when warmed. The solvent was then removed by rotary evaporation and after addition of 9-phenanthrene carbaldehyde (Aldrich) reaction run following the normal reductive amination procedure outlined in 18C to give 2-methyl-2-((9-phenanthrenylmethyl)amino)-1,4-butanediol methanesulfonate·3/10 $H_2O$ mp 174—176° (dec), ($EtOH/Et_2O$), (C, H, N, S).

Example 55

(1α, 2β, 3α)-2-((1-Pyrenylmethyl)amino)-1,3-cyclohexandiol methane sulfonate

55A. (1α, 2β, 3α)-2-Amino-1,3-cyclohexanediol acetate

This compound was prepared by the method of F. Lichtendthaler (*Ber.* 96, 851 (1963), mp 175—177°, (C, H, N), (lit. 178—179°, F. Lichtenthaler, *Ber.* 96, 845 (1963).

55B. (1α, 2β, 3α)-2-((1-Pyrenylmethyl)amino)-1,3-cyclohexanediol methanesulfonate

To a RB flask was added (1?, 2?, 3?)-2-amino-1,3-cyclohexanediol acetate (54A) and an equimolar amount of $NaOCH_3$ and $CH_3OH$ (100 mL). After warming, the solvent was removed by rotary evaporation, and after addition of 1-pyrenecarbaldehyde (Aldrich) the reaction run following the normal reductive amination procedure outlined in 18C to give (1α, 2β, 3α)-2-((1-pyrenylmethyl)amino)-1,3-cyclohexanediol methanesulfonate mp 257—258° (dec), ($CH_3OH/Et_2O$), (C, H, N, S).

Example 56

2-Isopropyl-2-((3-perylenylmethyl)amino)-1,3-propanediol

56A. 2-Isopropyl-2-nitro-1,3-propanediol

A solution of 2-methyl-1-nitropropane (38.7 g, 0.375 mol prepared by the procedure of N. Kornblum, B. Taube, and H. Ungnade, *J. Am. Chem. Soc.* 76, 3209 (1954) and $NEt_3$ (Eastman 3.79 g, 0.0375 mol) in $CH_3OH$ (50 mL) was added dropwise 37% aqueous formaldehyde solution (Mallinckrodt, 76.2 g, 0.938 mol), at a rate such that the reaction mixture temperature did not exceed 30°. After 72 h, the solution was concentrated under vacuum and the residue was dissolved in $H_2O$ (250 mL). The solution was continuously extracted for 1 h with $CH_2Cl_2$ (1 L). The $CH_2Cl_2$ solution was dried ($MgSO_4$), filtered, and concentrated to give 53.3 g (87%) of 2-isopropyl-2-nitro-1,3-propanedioll a waxy, white solid mp 67—72° (lit. mp 87—88°, B. M. Vanderbilt and H. B. Hass, *Ind. Eng. Chem.* 32, 34 (1940). In our hands this procedure failed to give the desired compound).

56B. 2-Amino-2-isopropyl-1,3-propanediol acetate

Using the procedure in 43E, 2-isopropyl-2-nitrol-1,3-propanediol gave a 98% yield of 2-amino-2-isopropyl-1,3-propanediol acetate mp 155—155.5°. H. S. Broadbent *et. al., J. Heterocyclic Chem.,* 13, 337 (1975) report the synthesis of this compound as the free base (mp 70—72°)).

56C. 2-Isoproyl-2-((3-perylenylmethyl)amino)-1,3-propanediol methanesulfonate $\frac{1}{2}$ $H_2O$

To a RB flask was added 2-amino-2-isopropyl-1,3-propanediol acetate·β $H_2O$ (56B) and an equimolar amount of $NaOCH_3$ (MCB) and $CH_3OH$ (100 mL). After warming, the solvent was removed by rotary evaporation and after addition of perylene-3-carbaldehyde (18A), the reaction run following the normal reductive amination procedure outlined in 18C to give 2-isopropyl-2-((3-perylenylmethyl)amino)-1,3-propanediol methanesulfonate·$\frac{1}{2}H_2O$ mp 189—191° (dec), ($EtOH/Et_2O$), (C, H, N, S).

Example 57

2-Methyl-2-((3-perylenylmethyl)amino)-1,4-butanediol methanesulfonate-3/10 $H_2O$

Using the reductive amination procedure described for 54G, perylene-3-carbaldehyde (18A) and 2-amino-2-methyl-1,4-butanediol hydrochloride (54F) gave 2-methyl-2-((3-perylmethyl)amino)-1,4-butanediol methanesulfonate·3/10 $H_2O$ mp 182—184° (dec), ($EtOH/Et_2O$), (C, H, N, S).

Example 58

(1α, 2β, 3α)-2-((3-perylenylmethyl)amino)-1,3-cyclhexanediol methanesulfonate

Using the reductive amination procedure outlined for 55B, perylene-3-carbaldehyde (18A) and (1α, 2β, 3α)-2-amino-1,3-cyclohexaendiol (55A) gave (1α, 2β, 3α)-2-amino-1,3-cyclohexanediol (55A) gave (1α,2β,3α)-2-((3-perylenylmethyl)amino)-1,3-cyclohexanediol methanesulfonate mp 237—240° (dec), ($CH_3OH/Et_2O$), (C, H, N, S).

Example 59

2-(((3-Ethyl-1-pyrenyl)methyl)amino)-2-methyl-1,3-propanediol

59A. 3-Ethylpyrene-1-carboxaldehyde

1-Ethylpyrene (Cambridge Chemical, Inc., 55 g, 0.239 mol) was formylated according to the procedure outlined in 1A, except that $CH_2Cl_2$ (500 mL) was used as the reaction solvent. The isomeric aldehyde poduct mixture (48.2 g, 0.187 mol) (which could not be purified by chromatography on $SiO_2$) was treated with $LiBH_4$ (Alfa-Ventron, 2 g, 0.09 mol) in THF (800 mL). After 12 h, the reaction mixture was poured into ice water and acidified to pH = 2 with 1 N HCl. The isomeric alcohol mixture isolated (48 g, 0.18 mol) was chromatographed by preparative HPLC using $CH_2Cl_2$ as the eluting solvent. A poorly resolved, 3-component band resulted. The first component to elute was isolated by front-shaving and purified by recycling. The purified component (8.5 g) was treated directly with $BaMnO_4$ (Aldrich, 15.75 g, 0.61 mol) in $CH_2Cl_2$ (400 mL). After 3 h of refluxing, the mixture was filtered and the solvent removed to give the crude aldehyde. This was purified by preparative HPLC using $CH_2Cl_2$ as the eluting solvent to give 4.38 g (7%) of 3-ethylpyrene-1-carboxaldehyde mp 134—135.5°, (C, H), ($CH_2Cl_2$/hexane).

59B. 2-(((3-Ethyl-1-pyrenyl)methyl)amino)-2-methyl-1,3-propanediol methanesulfonate·3/10 $H_2O$

Using the reductive amination procedure outlined in 18C, 3-ethylpyrenecarboxaldehyde (59A) and 2-methyl-2-amino-1,3-propanediol (Aldrich) gave 2-(((3-ethyl-1-pyrenyl)methyl)amino)-2-methyl-1,3-propanediol methanesulfonate·3/10 $H_2O$ mp 219—219° (dec), ($EtOH/Et_2O$), (C, H, N, S).

Example 60

2-Isopropyl-2-((9-phenanthrenylmethyl)amino)-1,3-propanediol methanesulfonate

Using the reductive amination procedure described for 56C, 9-phenanthrenecarbaldehyde (Aldrich) and 2-amino-2-isopropyl-1,3-propanediol acetate (56B) gave 2-isopropyl-2-((9-phenthrenylmethyl)amino)-1,3-propanediol methanesulfonate mp 192—194° (dec), ($EtOH/Et_2O$), (C, H, N, S).

Example 61

(1α,2β,3α)-2-((9-Phenanthrenylmethyl)amino)-1,3-cyclohexanediol methanesulfonate

Using the reductive amination procedure described for 56C, phenanthrene-9-carbaldehye (Aldrich) and (1α,2β,3α)-2-amino-1,3-cylohexanediol acetate (56B) gave 1α,2β,3α)-2-((9-phenanthrenylmethyl)amino)-1,3-cyclohexanediol methanesulfonate mp 222—223°, ($EtOH/Et_2O$), (C, H, N, S).

Example 62

2-Isoropyl-2-((1-pyrenylmethyl)amino)-1,3-propanediol hydrochloride

Using the reductive amination procedure described for 56C, pryene-1-carbaldehyde (Aldrich) and 2-amino-2-isopropyl-1,3-propanediol acetate (56B) gave 2-isopropyl-2-((1-pyrenylmethyl)amino-1,3-propanediol hydrochloride mp 244—245° (dec), ($CH_3OH/Et_2O$), (C, H, N, Cl).

Example 63

2-(((8-Ethoxy-1-pyrenyl)methyl)amino-2-methyl-1,3-propanediol

63A. 8-Ethoxypyrene-1-carboxaldehyde

Using the method of E. Profft and R. Biela, *Chem. Ber.,* 94, 2374-82, 1-ethoxypyrene (Cambridge Chemical, Inc., 25 g, 0.101 mol) yielded an insoluble red formylation complex that was filtered from the reaction mixture and hydrolyzed. The crude aldehyde obtained (mp 102—122°) was recystallized 2X from $CH_2Cl_2$/hexane to yield 7.52 g (27%) of 8-methoxypyrene-1-carboxaldehyde mp 135—136°, (lit 135.5—136.5°, E. Profft and R. Biela, *Chem. Ber.* 94, 2374—82), (C, H).

63B. 2-(((8-Ethoxy-1-pyrenyl)methyl)amino)2-methyl-1,3-propanediol methanesulfonate

Using the reductive amination procedure described in 18, 8-ethoxypyrene-1-carbaldehyde and 2-amino-2-methyl-1,3-propanediol (Aldrich) gave 2-(((8-ethoxy-1-pyrenyl)methyl)amino)-2-methyl-1,3-propanediol methanesulfonate mp 203—203° (dec), ($EtOH/Et_2O$), (C, H, N, S).

Antitumour Screening Results

Methods for evaluating the antitumour activity of these compounds are essentially those used in the Tumour Panel by the Development Therapeutics Program, Division of Cancer Treatment, National Cancer Institute, A. Goldin, *et al., Methods in Cancer Research,* Vol. XVI, p. 165, Academic Press (1979). Some modifications in dose level and schedule have been made to increase the testing efficiency.

Example 64

Lymphocytic Leukaemia P388/0 Test

CD2—$F_1$ mice, of the same sex, weighing 20 + 3 g, are used for this test. Control and test animals are injected intraperitoneally with the suspension of α$10^6$ viable P388/0 tumour cells on day 0. In each test, several dose levels which bracket the $LD_{20}$ of the compound are evaluated; each dose level group contains 6 animals. The test compounds are prepared either in physiologic saline containing 0.05% Tween 80 or distilled water containing 5% dextrose and are administered intraperitoneally on days 1, 5 and 9 relative to tumour implant. Doses are on a mg/kg basis according to individual animals' body weights. The day of death for each animal is recorded, the median identified for each group and the ratios of median survival time for treated (T)/control (C) groups are calculated. The criterion for activity is T/C × 100 ⩾ 120%. Results of P388/0 testing are summarized in Table I below.

TABLE I

P388/0 Screening Results

| Compound of Example No. | Optimal Dose (mg/kg) | T/C × 100% (Excluding 30 Day Survivors | $LD_{20}$$^A$ |
|---|---|---|---|
| 3A | 250 | +168 | 170 |
| 3B | 450 | +145 | 450 |
| 4 | 120 | +120 | 450 |
| 5 | 200 | +127 | 450 |
| 6A | 303 | +240 | 225 |
| 7 | 366 | +125 | 450 |
| 8 | 220 | +125 | 600 |
| 9 | 300 | +128 | 600 |
| 1A | 300 | +225 | 375 |
| 12 | 198 | +190 | 215 |
| 13 | 300 | +163 | 450 |
| 15 | 220 | +131 | 220 |
| 17 | 144 | +170 | 144 |
| 18B | 262 | +209 | 175 |
| 20B | 450 | +205 | (300) |
| 21C | 450 | +125 | (300) |
| 24B | 175 | +133 | 358 |
| 41B | 250 | +120 | 225 |
| 25 | 555 | +60 | (500) |
| 26 | 1013 | +185 | (675) |
| 28B | 250 | +135 | 557 |

TABLE I (continued)

P388/0 Screening Results

| Compound of Example No. | Optimal Dose (mg/kg) | T/C × 100% (Excluding 30 Day Survivors | $LD_{20}$[A] |
|---|---|---|---|
| 29 | 300 | +165 | (300) |
| 30C | 40 | +145 | 45 |
| 2 | 88 | +240 | 80 |
| 31F | 104 | +120 | 120 |
| 33D | 100 | +220 | 150 |
| 32B | 159 | +170 | 212 |
| 43G | 125 | +177 | 125 |
| 44 | 100 | +150 | 90 |
| 45C | 168 | +130 | 225 |
| 47E | 190 | +130 | 300 |
| 37H | 110 | +120 | 150 |
| 48C | 225 | +145 | 225 |
| 49D | 180 | +130 | 135 |
| 51 | 300 | +120 | (675) |
| 52 | 200 | +205 | 325 |
| 53 | 590 | +140 | (675) |
| 54G | 180 | +195 | 180 |
| 55B | 180 | +280 | 180 |
| 56C | 110 | +120 | (675) |
| 57 | 375 | +130 | 225 |

A. Values in parentheses are the highest non-toxic dose where the $LD_{20}$ was not determined.

Example 65

Lymphocytic Leukemia L1210 Test

The protocol for this test is identical to that for P388/0, except that the number of L1210 cells implanted on day 0 is $10^5$/mouse. The mouse CD2—$F_1$ strain is used, and the criterion for activity is T/C $\times$ 100 > 125%. Results of L1210 testing are summarized in Table II below.

TABLE II

Screening Results for L1210

| Compound No. | Dose (mg/kg) | T/C × 100% Excluding 30 day Survivors |
|---|---|---|
| 1A | 150 | +129 |

Example 66

Melanotic Melanoma B16

B6C3—$F_1$ mice of the same sex, weighing 20 $\pm$ 3 g, are used for this test. A suspension of B16 cells is prepared from a non-necrotic portion of solid tumour tissue obtained from a passage mouse. One gram of tumour is homogenized in 9 mL ice-cold Earle's salts solution and filtered through 1000 mesh screen to remove debris. 0.5 mL of the resulting brei is injected intraperitoneally into each animal. Dosing is carried out as in the P388/0 and L1210 tests. Days of death are recorded for a 60 day period, and T/C ratio calculated as in the P388/0 and L1210 tests. The criterion for activity is T/C $\times$ 100 > 125%. The results of B16 testing are summarized below in Table III.

TABLE III

Screening Results for B16 Melanoma

| Compound of Example No. | Dose (mg/kg) | T/C × 100% Excluding 60 day Survivors |
|---|---|---|
| 6A | 225 | +159 |
| 12 | 150 | +133 |
| 18B | 275 | +173 |
| 2 | 70 | +125 |
| 20B | 300 | +167 |

Example 67

Lewis Lung Carcinoma Test

This tumour arose spontaneously in the lung of a C57B1/6 mouse and is maintained by subcutaneous passage in that strain. The solid tumour is excised aseptically and placed in sterile saline. Pieces of viable tumour tissue are minced finely with scissors and forced through a 200 mesh stainless steel screen to disaggregate the tumour cells into a suspension. $10^6$ Viable cells are injected intravenously into the tail vein of BD—$F_1$, mice of the same sex weighing 20—3 g. In each test, several dose levels which bracket the $LD_{20}$ for the compound are evaluated. Ten animals are included in each dose level group, and twenty animals in the untreated control group. The test compounds are prepared and administered as in the P388/0 protocol. The day of death for each animal is recorded, the median identified for each group and the ratios of median survival time for treated (T)/control (C) groups are calculated. The criterion for activity is T/C $\times$ 100 > 140%. The results of Lewis Lung testing are summarized in Table IV.

TABLE IV

Screening Results for Lewis Lung

| Compound No. | Dose (mg/kg) | T/C × 100% Excluding 60 day Survivors |
|---|---|---|
| 1B | 150 | 108 |

Example 68

*Herpes simplex* 1/vero Test

Antiviral testing against *Herpes simplex* 1/vero was done using plaque inhibition methods as outlined in P. Collins and D. J. Bauer, *Proc. N. Y. Acad. Sci.* 284, 49 (1977) and by plaque reduction methods as outlined in P. Collins and D. J. Bauer, *J. Antimicrobial Chemotherapy* 3, *Supplement A,* 73, (1977). The column headings labeled Score, Toxicity, and Zone of Inhibition refer to the plaque inhibition screen while the $IC_{50}$ heading to the plaque reduction screen.

TABLE V

Results of Antiviral Screening Against *Herpes simplex* I/vero

| Compound Example No. | Score[A] | Toxicity | Zone of Inhibition[B] | $IC_{50}$[B] |
|---|---|---|---|---|
| 7 | −2 | Y | | |
| 11 | −2 | Y | | |
| 17 | −3 | Y | | |
| 24B | −3 | Y | | 17.25 (ST at 25) |
| 41B | −4 | Y | | 20 (T) |
| 42C | −4 | Y | | 6.0 |
| 26 | −4 | Y | | 45 |
| 29 | −3 | Y | 30 (ST 19) | |
| 33D | −3 | Y | 40 (VT at 28) | |
| 32B | −2 | Y | | |
| 34 | −3 | Y | | 6.10 |
| 44 | −4 | Y | | 3.6 |
| 46 | −2 | Y | 32 (ST at 20) | |
| 36D | −2 | Y | 32 (ST at 20) | |
| 50 | −4 | Y | 35 (T25) | |

A. Score code:
- 0 = no inhibition
- −1 = 1—25% inhibition
- −2 = 26—50% inhibition
- −3 = 51—75% inhibition
- −4 = 76—100% inhibition

B. ST = slight toxicity, T = toxic, VT = very toxic

Antibacterial Screening

Antibacterial testing against *Mycoplasma smegmatis* (S3264) and *Streptococcus pyogenes* (CN10) was done with slight modications using standard agar dilution assays as outlined in *Manual of Clinical Microbiology, Second Ed.,* E. H. Lennette, E. H. Spaulding and J. P. Traunt, Eds., American Society for Microbiology, Washington, DC, 1974.

Example 69
*Mycoplasma smegmatis* Test

TABLE VI

Results of Antibacterial Screening Against
*Mycoplasma smegmatis* (S3264)

| Compound Example No. | MIC (mg/L) |
|---|---|
| 3A | ≤5 |
| 4 | <5 |
| 6A | 3 |
| 7 | 10 |
| 8 | ≤5 |
| 9 | 10 |
| 1A | ≤5 |
| 10 | 10 |
| 11 | >50 |
| 12 | >50 |
| 13 | 10 |
| 14 | >50 |
| 15 | 5 |
| 16 | 10 |
| 17 | ≤5 |
| 25 | ≤10 |
| 29 | ≤10 |
| 30C | 10 |
| 2 | 10 |
| 33D | 10 |

Example 70

*Streptococcus pyogenes* Testing

TABLE VII

Results of Antibacterial Testing Against *Streptococcus pyogenes* (CN10)

| Compound of Example No. | MIC (mg/L) |
|---|---|
| 3A | 10 |
| 4 | 10 |
| 6A | 3 |
| 10 | 10 |
| 11 | 10 |
| 12 | 5 |
| 13 | 5 |
| 15 | 5 |
| 16 | ≤5 |
| 29 | 30 |
| 2 | ≤3 |
| 33D | 10 |

Example 71

*Candida albicans* Testing

Antifungal testing against *Candida albicans* (CN1863) was done with slight modifications using a combination of broth and sugar and agar dilution assays as outlined in *Laboratory Handbook of Medical Mycology,* Chapter 6, pages 441—446, M. R. McGinnis, Academic Press, New York, NY, 1980.

TABLE VIII

Results of Antifungal Screening Against *Candida albicans* (CN1863)

| Compound of Example No. | MIC (mg/L) |
|---|---|
| 3A | 50 |
| 4 | 10 |
| 6A | 30 |
| 7 | 50 |
| 8 | 50 |
| 9 | 50 |
| 1A | 50 |
| 10 | 50 |
| 11 | 10 |
| 12 | >50 |
| 13 | 50 |
| 14 | >50 |
| 15 | 10 |
| 16 | 50 |
| 17 | >50 |
| 30C | 30 |
| 2 | 30 |
| 33D | 30 |

Medium: Wellcotest® sensitivity test agar plus 7% lysed horse blood.

Example 72

*Trichomonas vaginalis* Testing

Antiprotozoal testing against *Trichomonas vaginalis* was done using methods outlined by R. M. Michaels in *Advances in Chemotherapy* 3, 39—108 (1968).

TABLE IX

Results of Antiprotozoal Testing Against *Trichomonas vaginalis (in vitro)*

| Compound of Example No. | Dose (mg/L) | Result[A] |
|---|---|---|
| 3A | 40 | −3 |
| 4 | 6.25 | −4 |
| 6A | 40 | −3 |
| 7 | 40 | −4 |
| 1A | 40 | −4 |
| 10 | 40 | −2 |
| 13 | 40 | −4 |
| 11 | 40 | −4 |

(Stenton or Modified Diamond's medium)

A. Screen Code

0 = no inhibition
−1 = 1—25% inhibition
−2 = 26—50% inhibition
−3 = 51—75% inhibition
−4 = 76—100% inhibition

Example 73

*Nippostrongylus brasiliensis* Testing

Anthelmintic testing against *Nippostrongylus brasiliensis* was done using methods as outlined in D. C. Jenkins, R. Armitage, and T. S. Carrington, *Zeitschrift fur Parasitenkunde 63,* 261—269 (1980).

TABLE X

Results of Anthelmintic Screening Against
*Nippostrongylus brasiliensis*
(Immature — free living stages)

| Compound of Example No. | MIC (mg/L) |
|---|---|
| 3A | 10 |
| 4 | 1 |
| 5 | 10 |
| 6A | 100 |
| 1A | 50 |
| 10 | 50 |
| 17 | ≥50 |
| 16 | 50 |
| 15 | 50 |
| 13 | ≥50 |
| 12 | ≥50 |
| 11 | 50 |

Example 74
*Eimeria tenella* Testing

Antiprotozoal testing against *Eimeria tenella* was done using methods outlined in V. S. Latter and D. Wilson, *Parasitology 79, 169 (1979).*

TABLE XI

Results of Antiprotozoal Screening Against
*Eimeria tenella (in vitro)*

| Compound of Example No. | Dose (mg/L) | Result[A] |
|---|---|---|
| 3A | 5 | −2 |
| 17 | 5 | −4 |
| 16 | 0.31 | −2 |
| 15 | 5 | −2 |
| 14 | 5 | −4 |
| 13 | 5 | −4 |
| 12 | 0.37 | −4 |
| 11 | 5 | −3 |

A. Screen Code
0 = no inhibition
−1 = 1—25% inhibition
−2 = 26—50% inhibition
−3 = 51—75% inhibition
−4 = 76—100% inhibition

Example 75
*Brugia pahangi* Testing

Anthelmintic testing against *Brugia pahangi* was done using methods outlined in D. A. Denham *et al., Transactions of the Royal Society of Tropical Medicine and Hygiene 73,* 673—676 :1979).

TABLE XII

Results of Screening Against *Brugia pahangi* microfilaria *(in vitro)*

| Compound of Example No. | Dose (mg/L) | Result[A] | MIC (mg/L) |
|---|---|---|---|
| 3A | 3.1 | −4 | 3.10 |
| 4 | 0.78 | −4 | 0.78 |
| 5 | 12.5 | −2 | |
| 14 | 12.5 | −2 | |

A. Screen Code
0 = no inhibition
−1 = 1—25% inhibition
−2 = 26—50% inhibition
−3 = 51—75% inhibition
−4 = 76—100% inhibition

Example 76
$LD_{50}$ Tests

TABLE XIII

$LD_{50}$ Values for Selected Compounds
(IP single dose — $CD_1$ Male Mouse)

| Compound of Example No. | $LD_{50}$ (mg/kg) |
|---|---|
| 6aA | 160 |
| 7 | 350 |
| 1A | 350 |
| 10 | 350 |
| 17 | 160 |
| 16 | 130 |
| 15 | 160 |
| 13 | 250 |
| 12 | 160 |
| 11 | 410 |

**Claims**

1. A compound of the formula (I)

$$ArCH_2R^1 \qquad (I)$$

or a monomethyl or monoethyl ether thereof, the compound of formula (I) including its ethers containing no more than 30 carbon atoms in total, or an ester or salt thereof wherein:

Ar is perylene, fluoranthene, chrysene, pyrene or triphenylene, optionally substituted by one or two substituents, or when Ar is anthracene or phenanthrene optionally substituted by one, two or three substituents; said substituents containing not more than four carbon atoms in total when taken together being the same or different and are selected from halogen; cyano; $C_{1-4}$ alkyl or $C_{1-4}$ aloxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0, 1 or 2 and $R^2$ is $C_{1-2}$ alkyl optionally substituted by hydroxy or $C_{1-2}$ alkoxy; or Ar is optionally substituted by a group $NR^3R_4$ containing not more than 5 carbon atoms wherein $R^3$ and $R^4$ are the same or different and each is a $C_{1-3}$ alkyl group or $NR^3R^4$ forms a five- or six-membered heterocyclic ring optionally containig one or two additional heteroatoms;

$R^1$ contains not more than eight cabon atoms and is a group

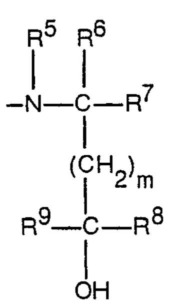

or

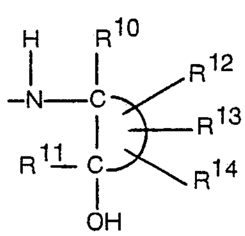

wherein

m is 0 or 1;

$R^5$ is hydrogen or methyl;

$R^6$ and $R^7$ are the same or different and each is hydrogen or $C_{1-3}$ alkyl optionally substituted by hydroxy;

$R^8$ and $R^9$ are the same or different and each is hydrogen or $C_{1-3}$ alkyl;

—C—C— is a five- or six-membered saturated carbocyclic ring

$R^{10}$ is hydrogen, methyl or hydroxymethyl;

$R^{11}$, $R^{12}$ and $R^{13}$ are the same or different and each is hydrogen or methyl;

$R^{14}$ is hydrogen, methyl, hydroxy, or hydroxymethyl;

and provided that when Ar is 6-chrysenyl, 3- or 7-fluoranthenyl, 2-triphenylenyl, or 1- or 9-anthracenyl optionally substituted by one or two substituents as hereinbefore defined; other than optionally substituted butyl or butoxy;

$R^6$ is $C_{1-3}$ alkyl optionally substituted by hydroxy;

$R^7$ is hydrogen, $C_{1-3}$ alkyl or hydroxymethyl;

$R^8$ to $R^{12}$ and $R^{14}$ are as hereinbefore defined;

$R^{13}$ is hydrogen; and m is 0; then $R^5$ is methyl and the compound N-(2-hydroxyethy)-N-methyl- 9-aminomethylanthracene being excluded.

2. A compound according to claim 1 wherein Ar is 6-chrysenyl, 3- or 7-fluoranthenyl, 2-triphenylenyl, 1- or 9-anthracenyl, 3-perylenyl, 9-phenanthrenyl or 1-pyrenyl.

3. A compound according to either claim 1 or claim 2 wherein $R^1$ is

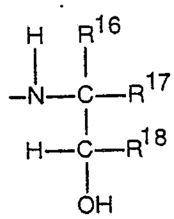 or 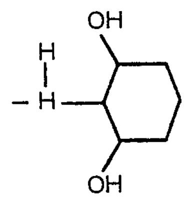

wherein $R^{16}$ is $CH_2OH$, $CH(CH_3)OH$ or $CH_2CH_2OH$,

$R^{17}$ is hydrogen, $C_{1-3}$ alkyl or $CH_2OH$,

$R^{18}$ is hydrogen or methyl.

4. A compound according to any one of claims 1 to 3 wherein $R^1$ is

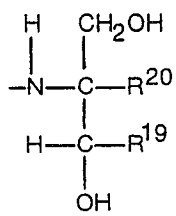

wherein $R^{19}$ is hydrogen or methyl and $R^{20}$ is hydrogen, methyl or ethyl.

5. A compound according to any one of claims 1 to 4 selected from:

2-Methyl-2-((1-pyrenylmethyl)amino)propanol
3-((1-Pyrenylmethyl)amino)propanol
3-Methyl-2-((1-pyrenylmethyl)amino)butanol
2-Ethyl-2-((1-pyrenylmethyl)amino)-1,3-propanediol
2-((1-Pyrenylmethyl)amino)butanol
2-((1-Pyrenylmethyl)amino)propanol
1-((1-pyrenylmethyl)amino)-2-propanol
2-Methyl-2-((1-pyrenylmethyl)amino)-1,3-propanediol
1-((1-Pyrenylmethyl)amino)-2-butanol
2-(N-(1-Pyrenylmethyl)methylamino)ethanol
2-Hydroxymethyl-2-((1-pyrenylmethyl)amino)-1,3-propanediol
2-((1-Pyrenylmethyl)amino-1,3-propanediol
2-(((1-Pyrenylmethyl)amino)methyl)-2-propanol
2-((1-Pyrenylmethyl)amino)ethanol
4-((1-Pyrenylmethyl)amino)-2-butanol
2-Methyl-2-((9-phenanthrenylmethyl)amino)-1,3-propanediol
2-Methyl-2-((3-Perylenylmethyl)amino)-1,3-propanediol
2-Methyl-2-((4-phenanthrenylmethyl)amino)-1,3-propanediol
2-Methyl-2-(((4,5,10-trichloro-9-anthracenyl)methyl)amino)-1,3-propanediol
2-(((10-chloro-2,3-dimethyl-9-anthracenyl)methyl)amino)-2-methyl-1,3-propanediol
2-(((2-tert-Butyl-10-chloro-9-anthracenyl)methyl)amino)-2-methyl-1,3-propanediol
2-(((3-tert-Butyl-10-chloro-9-anthracenyl)methyl)amino)-2-methyl-1,3-propanediol

2-Methyl-2-((2-phenanthrenylmethyl)amino)-1,3-propanediol
2-Methyl-2-((3-phenanthrenylmethyl)amino)-1,3-propanediol
2-((2-Anthracenylmethyl)amino)-2-methyl-1,3-propanediol
2-Methyl-2-(((2,6,10-trichloro-9-anthracenyl)methyl)amino)-1,3-propanediol
2-(((6-Bromo-1-pyrenyl)methyl)amino)-2-methyl-1,3-propanediol and
2-(((8-Bromo-1-pyrenyl)methyl)amino)-1,3-propanediol
2-Methyl-2-((4-pyrenylmethyl)amino)-1,3-propanediol
2-((6-Chrysenylmethyl)amino)-2-methyl-1-propanol
2-(((10-Butoxy-9-anthracenyl)methyl)amino)-2-methyl-1,3-propanediol
2-(((10-Butyl-9-anthracenyl)methyl)amino)-2-methyl-1,3-propanediol
2-((3-Fluoranthenylmethyl)amino)-2-methyl-1-propanol
2-((8-Fluoranthenylmethyl)amino)-2-methyl-1,3-propanediol
(±)(2R*,3S*)-2-((1-Pyrenylmethyl)amino)-2-methyl-1,3-butanediol
(±)(2R*,3S*)-2-((9-Phenanthrenylmethyl)amino)-2-methyl-1,3-butanediol
2-(((3-chloro-8-fluoranthenyl)methyl)amino)-2-methyl-1,3-propanediol and
2-(((4-Chloro-8-fluoranthenyl)methyl)amino)-2-methyl-1,3-propanediol
2-Ethoxymethyl-2-((1-pyrenylmethyl)amino)-1,3-propanediol
2-Ethoxymethyl-2-((9-phenanthrenylmethyl)amino)-1,3-propanediol
2-(((10-Ethoxy-9-phenanthrenyl)methyl)amino)-2-methyl-1,3-propanediol
2-(N-((6-Chrysenylmethyl)-N-methylamino)-2-methyl-1,3-propanediol
2-((3-Chrysenylmethyl)amino)-2-methyl-1,3-propanediol
2-((2-Chrysenylmethyl)amino)-2-methyl-1,3-propanediol
2-(N-(3-Fluoranthenylmethyl)-N-methylamino)-2-methyl-1,3-propanediol
3-Methoxy-2-methyl-2-((1-pyrenylmethyl)amino)-1-propanol
3-Methoxy-2-methyl-2-((9-phenanthrenylmethyl)amino)-1-propnaol
2-(((3-Ethyl-8-fluoranthenyl)methyl)amino)-2-methyl-1,3-propanediol
1-(((4-Ethyl-8-fluoranthenyl)methyl)amino)-2-methyl-1,3-propanediol
2-(((9-Ethoxy-1-phenanthrenyl)methyl)amino)-2-methyl-1,3-propanediol
2-((3-Perylenylmethyl)amino)-3-methoxy-2-methyl-1-propanol
(±)(2R*,3S*)-2-((3-Perylenylmethyl)amino)-2-methyl-1,3-butanediol
2-((3-Perylenylmethyl)amino)-2-ethoxymethyl-1,3-propanediol
2-Methyl-2-((9-phenanthrenylmethyl)amino)-1,4-butanediol
(12β,3α)-2-((1-Pyrenylmethyl)amino)-1,3-cyclohexanediol
2-Isopropyl-2-((3-perylenylmethyl)amino)-1,3-propanediol
2-Methyl-2-((3-perylenylmethyl)amino-1,4-butanediol
(1α,2β,3α)-2-((3-Perylenylmethyl)amino)-1,3-cyclohexanediol
2-(((3-Ethyl-1-pyrenyl)methyl)amino)-2-methyl-1,3-propanediol
2-Isopropyl-2-((9-phenanthrenylmethyl)amino)-1,3-propanediol
1-(1α,2β,3α)-2-((9-Phenanthrenylmethyl)amino)-1,3-cyclohexanediol
2-Isoproply-2-((1-pryenylmethyl)amino)-1,3-propanediol
2-(((8-Ethoxy-1-pyrenyl)methyl)amino)-2-methyl-1,3-propanediol

and ethers, esters and salts thereof.

6. A hydrochloride, methanesulphonate, ethanesulphonate, lactate, citrate or isethionate salt of a compound according to any one claim 1 to 5.

7. A pharmaceutical formulation comprising a compound according to any one of claims 1 to 6 herein together with a pharmaceutically acceptable carrier therefor.

8. A pharmaceutical formulation according to claim 7 suitable for parental administration comprising a sterile aqueous preparation of a compound according to any one of claims 1 to 6 herein which is isotonic with the blood of the recipient.

9. A method for the preparation of a pharmaceutical formulation according to either claim 7 or 8 which comprises bringing into association a compound of formula (I) or ether, ester or pharmaceutically acceptable salt thereof and pharmaceutically acceptable carrier thereof.

10. A compound according to any one of claims 1 to 6 for use in medicine.

11. A method for the preparation of a compound according to any one of claims 1 to 6 which comprises:

1) The reduction of a compound of formula (II)

($R^1$ when $R^5$ = H)

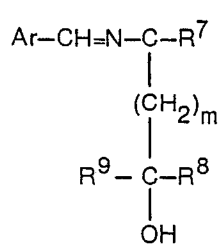

(II)

wherein $R^1$—$R^4$ are as hereinbefore defined or an appropriately protected derivative thereof followed by deprotection where appropriate;

2) the reduction of a compound of the formula (V)

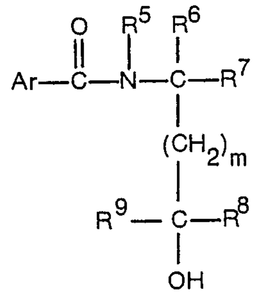

(V)

wherein Ar and $R^1$—$R^4$ are as hereinbefore defined and the hydroxy groups are optionally protected, followed by deprotection of the hydroxy groups where appropriate;

3) The reaction of a compound $ArCH_2L$ (wherein Ar is as hereinbefore defined and L is a leaving group), with a compound of the formula (IV)

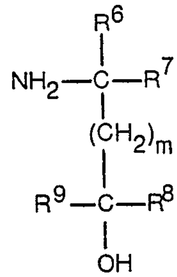

(where $R^5 = H$) (IV)

wherein Ar and $R^1$—$R^4$ are as defined in (I) except that $R^5 = H$.

12. Chemical intermediates being 9-anthracenyl carbaldehydes substituted by one, two or three substituents, containing not more than four carbon atoms in total when taken together being the same or different and being selected from $C_{1-4}$ alkyl or $C_{1-4}$ alkoxy each optionally substituted by hydroxy or $C_{1-2}$ alkoxy.

**Patentansprüche**

1. Verbindung der Formel (I)

$$ArCH_2R^1 \quad (I)$$

oder ein Monomethyl- oder Monoethylether davon, wobei die Verbindung der Formel (I) einschließlich ihrer Ether insgesamt nicht mehr als 30 Kohlenstoffatome enthält, oder ein Ester oder Salz davon;

worin Ar Perylen, Fluoranthen, Chrysen, Pyren oder Triphenyl ist, die gegebenenfalls durch einen oder zwei Substituenten substituiert sind, oder wenn Ar Anthracen oder Phenanthren ist, gegebenenfalls durch einen, zwei oder drei Substituenten substituiert sind, wobei die Substituenten insgesamt, wenn zusammengenommen, nicht mehr als vier Kohlenstoffatome enthalten und gleich oder verschieden sind und aus den folgenden ausgewählt werden; Cyano; $C_{1-4}$ Alkyl oder $C_{1-4}$ Alkoxy, die gegebenenfalls durch Hydroxy oder $C_{1-2}$ Alkoxy substituiert sind; Halogen substituiertes $C_{1-2}$ Alkyl oder $C_{1-2}$ Alkoxy; eine Gruppe $S(O)_nR^2$, worin n eine Zahl 0, 1 oder 2 ist und $R^2$ $C_{1-2}$ Alkyl ist, das gegebenenfalls durch Hydroxy oder $C_{1-2}$ Alkoxy substituiert ist; oder Ar ist gegebenenfalls durch eine Gruppe $NR^3R^4$ substituiert, die nicht mehr als 5 Kohlenstoffatome enthält, worin $R^3$ und $R^4$ gleich oder verschieden sind und jeweils eine $C_{1-3}$ Alkylgruppe bedeuten, oder $NR^3R^4$ bildet einen 5- oder 6-gliedrigen heterocyclischen Ring, der gegebenenfalls eines oder zwei zusätzliche Heteroatome enthält;

$R^1$ enthält nicht mehr als 8 Kohlenstoffatome und ist eine der folgenden Gruppen:

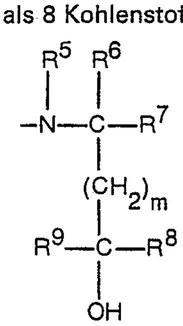

oder

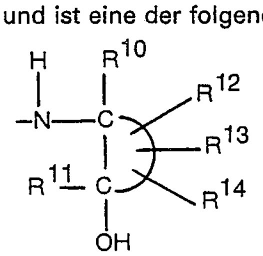

worin 0 oder 1 ist;

$R^5$ ist Wasserstoff oder Methyl;

$R^6$ und $R^7$ sind gleich oder verschieden und sind jeweils Wasserstoff oder $C_{1-3}$ Alkyl, das gegegenenfalls durch Hydroxy substituiert ist; $R^8$ und $R^9$ sind gleich oder verschieden und sind jeweils Wasserstoff oder $C_{1-3}$ Alkyl;

—C⌒C— ist eine 5- oder 6-gliedriger gesättigter carbocyclischer Ring; $R^{10}$ ist Wasserstoff, Methyl oder Hydroxymethyl;

$R^{11}$, $R^{12}$, und $R^{13}$ sind gleich oder verschieden und sind jeweils Wasserstoff oder Methyl;

$R^{14}$ ist Wasserstoff, Methyl, Hydroxy oder Hydroxymethyl; und mit der Maßgabe daß, wenn Ar 6-Chrysenyl, 3- oder 7-Fluoranthenyl, 2-Triphenylenyl, oder 1- oder 9-Anthracenyl, gegebenenfalls substituiert durch einen oder zwei Substituenten, wie obenstehend definiert, ist;

verschieden von gegebenfalls substituiertem Butyl oder Butoxy; $R^6$ $C_{1-3}$ Alkyl ist, gegebenfalls durch Hydroxy substituiert; $R^7$ Wasserstoff, $C_{1-3}$ Alkyl oder Hydroxymethyl ist; $R^8$ bis $R^{12}$ und $R^{14}$ die vorhin gegebene Bedeutung aufweisen; $R^{13}$ Wasserstoff ist und m 0 ist, dann ist $R^5$ Methyl; und wobei die Verbindung N-(2-Hydroxyethyl)-N-methyl-9-aminomethylanthracen ausgeschlossen ist.

2. Verbindung nach Anspruch 1, worin Ar 6-Chrysenyl, 3- oder 7-Fluoranthenyl, 2-Triphenylenyl, 1- oder 9-Anthracenyl, 3-Perylenyl, 9-Phenanthrenyl oder 1-Pyrenyl ist.

3. Verbindung nach einem der Ansprüche 1 oder 2, worin $R^1$ eine der folgenden Gruppen ist;

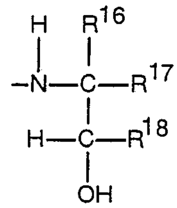

oder

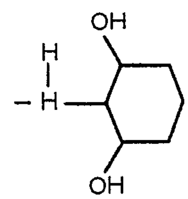

worin $R^{16}$ $CH_2OH$, $CH(CH_3)OH$ oder $CH_2CH_2OH$ ist,
$R^{17}$ Wasserstoff, $C_{1-3}$ Alkyl oder $CH_2OH$ ist,
$R^{18}$ Wasserstoff oder Methyl ist.

4. Verbindung nach einem der Ansprüche 1 bis 3, worin $R^1$ die folgende Gruppe ist:

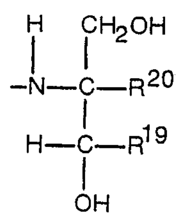

worin $R^{19}$ Wasserstoff oder Methyl und $R^{20}$ Wasserstoff, Methyl oder Ethyl ist.

5. Verbindung nach einem der Ansprüche 1 bis 4, ausgewählt aus:
2-Methyl-2-((1-pyrenylmethyl)amino)propanol
3-((1-Pyrenylmethyl)amino)propanol
3-Methyl-2-((1-pyrenylmethyl)amino)butanol
2-Ethyl-2-((1-pyrenylmethyl)amino)-1,3-propandiol
2-((1-Pyrenylmethyl)amino)butanol
2-((1-Pyrenylmethyl)amino)propanol
1-((1-Pyrenylmethyl)amino)-2-propanol
2-Methyl-2-((1-pyrenylmethyl)amino)-1,3-propandiol
1-((1-Pyrenylmethyl)amino)-2-butanol
2-(N-(1-Pyrenylmethyl)methylamino)ethanol
2-Hydroxymethyl-2-((1-pyrenylmethyl)amino)-1,3-propandiol
2-((1-Pyrenylmethyl)amino-1,3-propandiol
2-(((1-Pyrenylmethyl)amino)methyl)-2-propanol
2-(((10-Butyl-9-anthracenyl)methyl)amino)-2-methyl-1,3-propandiol
2-((3-Fluoranthenymethylamino)-2-methyl-1-propanol
2-((8-Fluoranthenylmethyl)amino)-2-methyl-1,3-propandiol
(±)(2R*,3S*)-2-((1-Pyrenylmethyl)amino)-2-methyl-1,3-butandiol
(±)(2R*,3S*)-2-((9-Phenanthrenylmethyl)amino)-2-methyl-1,3-butandiol
2-(((3-Chlor-8-fluoranthenyl)methyl)amino)-2-methyl-1,3-propandiol und
2-(((4-Chlor-8-fluoranthenyl)methyl)amino)-2-methyl-1,3-propandiol
2-Ethoxymethyl-2-((1-pyrenylmethyl)amino)-1,3-propandiol
2-Ethoxymethyl-2-((9-phenanthrenylmethyl)amino)-1,3-propandiol

2-(((10-Ethoxy-9-phenanthrenyl)methyl)amino)-2-methyl-1,3-propandiol
2-(N-((6-Chrysenylmethyl)-N-methylamino)-2-methyl-1,3-propandiol
2-((3-Chrysenylmethyl)amino)-2-methyl-1,3-propandiol
2-((2-Chrysenylmethyl)amino)-2-methyl-1,3-propandiol
2-(N-(3-Fluoranthenylmethyl)-N-methylamino)-2-methyl-1,3-propandiol
3-Methoxy-2-methyl-2-((1-pyrenylmethyl)amino)-1-propanol
3-Methoxy-2-methyl-2-((9-phenanthrenylmethyl)amino)-1-propanol
2-(((3-Ethyl-8-fluoranthenyl)methyl)amino)-2-methyl-1,3-propandiol
1-(((4-Ethyl-8-fluoranthenyl)methyl)amino)-2-methyl-1,3-propandiol
2-(((9-Ethoxy-1-phenanthrenyl)methyl)amino)-2-methyl-1,3-propandiol
2-((1-Pyrenylmethyl)amino)ethanol
4-((1-Pyrenylmethyl)amino)-2-butanol
2-Methyl-2-((9-phenanthrenylmethyl)amino)-1,3-propandiol
2-Methyl-2-((3-Perylenylmethyl)amino)-1,3-propandiol
2-Methyl-2-((4-phenanthrenylmethyl)amino)-1,3-propandiol
2-Methyl-2-(((4,5,10-trichlor-9-anthracenyl)methyl)amino)-1,3-propandiol
2-(((10-Chlor-2,3-dimethyl-9-anthracenyl)methyl)amino)-2-methyl-1,3-propandiol
2-(((2-tert-Butyl-10-chlor-9-anthracenyl)methyl)amino)-2-methyl-1,3-propandiol
2-(((3-tert-Butyl-10-chlor-9-anthracenyl)methyl)amino)-2-methyl-1,3-propandiol
2-Methyl-2-((2-phenanthrenylmethyl)amino)-1,3-propandiol
2-Methyl-2-((3-phenanthrenylmethyl)amino)-1,3-propandiol
2-((2-Anthracenylmethyl)amino)-2-methyl-1,3-propandiol
2-Methyl-2-(((2,6,10-trichlor-9-anthracenyl)methyl)amino)-1,3-propandiol
2-(((6-Brom-1-pyrenyl)methyl)amino)-2-methyl-1,3-propandiol und
2-(((8-Brom-1-pyrenyl)methyl)amino)-1,3-propandiol
2-Methyl-2-((4-pyrenylmethyl)amino)-1,3-propandiol
2-((6-Chrysenylmethyl)amino)-2-methyl-1-propanol
2-(((10-Butoxy-9-anthracenyl)methyl)amino)-2-methyl-1,3-propandiol
2-((3-Perylenylmethyl)amino)-3-methoxy-2-methyl-1-propanol
(±)(2R*,3S*)-2-((3-Perylenylmethyl)amino)-2-methyl-1,3-butandiol
2-((3-Perylenylmethyl)amino)-2-ethoxymethyl-1,3-propandiol
2-Methyl-2-((9-phenanthrenylmethyl)amino)-1,4-butandiol
(1α,2β,3α)-2-((1-Pyrenylmethyl)amino)-1,3-cyclohexandiol
2-Isopropyl-2-((3-perylenylmethyl)amino)-1,3-propandiol
2-Methyl-2-((3-perylenylmethyl)amino-1,4-butandiol
(1α,2β.3α)-2-((3-Perylenylmethyl)amino)-1,3-cyclohexandiol
2-(((3-Ethyl-1-pyrenyl)methyl)amino)-2-methyl-1,3-propandiol
2-Isopropyl-2-((9-phenanthrenylmethyl)amino)-1,3-propandiol
(1α,2β.3α)-2-((9-Phenanthrenylmethyl)amino)-1,3-cyclohexandiol
2-Isopropyl-2-((1-pyrenylmethyl)amino)-1,3-propandiol
2-(((8-Ethoxy-1-pyrenyl)methyl)amino)-2-methyl-1,3-propandiol
und Ether, Ester und Salze davon.

6. Ein Hydrochlorid-, Methansulfonat-, Ethansulfonat-, Lactat-, Zitrat- oder Isethionatsalz einer Verbindung nach einem der Ansprüche 1 bis 5.

7. Pharmazeutische Formulierung, enthaltend eine Verbindung nach einem der Ansprüche 1 bis 6 zusammen mit einem dafür pharmazeutisch annehmbaren Trägermaterial.

8. Pharmazeutische Formulierung nach Anspruch 7, die für die parenterale Verabreichung geeignet ist, enthaltend eine sterile, wässrige Zubereitung einer Verbindung nach einem der Ansprüche 1 bis 6, die isotonisch mit dem Blut des Empfängers ist.

9. Verfahren zur Herstellung einer pharmazeutischen Formulierung nach Anspruch 7 oder 8, bei dem man eine Verbindung der Formel (I) oder einen Ether, Ester oder pharmazeutisch annehmbares Salz davon mit einem dafür pharmazeutisch annehmbaren Trägermaterial in Kontakt bringt.

10. Verbindung nach einem der Ansprüche 1 bis 6 zur Verwendung in der Medizin.

11. Verfahren zur Herstellung einer Verbindung nach einem der Ansprüche 1 bis 6, bei dem man:

1) eine Verbindung der Formel (II) reduziert

($R^1$ wenn $R^5 = H$)

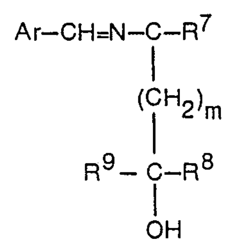

(II)

worin $R^1$ bis $R^4$ die vorhin gegebene Bedeutung aufweisen oder ein entsprechend geschütztes Derivat davon, gefolgt von einer Entfernung des Shutzes, wo es angebracht ist.

2. Eine Verbindung der Formel (V) reduziert

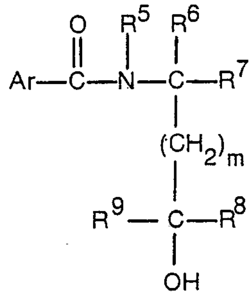

(V)

worin Ar und $R^1$ bis $R^4$ die vorhin gegebene Bedeutung aufweisen und die Hydroxygruppen gegebenenfalls geschützt sind, gefolgt von einer Entfernung des Schutzes von den Hydroxygruppen, wo es angebracht ist.

3. Eine Verbindung $ArCH_2L$ (worin Ar die vorhin gegebene Bedeutung aufweist und L eine austretende Gruppe ist) mit einer Verbindung der Formel (IV) umsetzt.

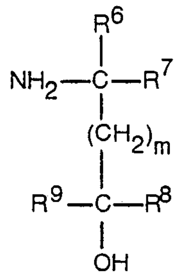

(wo $R^5 = 5$)

(IV)

worin Ar und $R^1$ bis $R^4$ die in (I) gegebene Bedeutung aufweisen, mit der Ausnahme, daß $R^5 = H$.

12. Chemische Zwischenprodukte, die 9-Anthracenylcarbaldehyde sind, die durch einen, zwei oder drei Substituenten substituiert sind, die insgesamt — wenn zusammengenommen —, nicht mehr als 4 Kohlenstoffatome enthalten und die gleich oder verschieden sind und aus $C_{1-4}$ Alkyl oder $C_{1-4}$ Alkoxy, jeweils gegebenfalls durch Hydroxy oder $C_{1-2}$ Alkoxy substituiert, ausgewählt sind.

**Revendications**

1. Composé de formule (I)

$$ArCH_2R^1 \qquad (I)$$

ou un éther monométhylique ou monoéthylique de celui-ci, le composé de formule (I) incluant ses éthers ne contenant pas plus de 30 atomes de carbone au total, ou un ester ou un sel de ce composé;

où Ar représente pérylène, fluoranthène, chrysène, pyrène ou triphénylène éventuellement substitué par un ou deux substituants, ou bien où Ar représente anthracène ou phénanthrène éventuellement substitué par un, deux ou trois substituants; ces substituants ne comptant pas plus de quatre atomes de carbone au total lorsqu'ils sont pris ensemble et étant identiques ou différents et choisis parmi halogéno; cyano; $C_{1-4}$alcoyle ou $C_{1-4}$alcoxy, chacun éventuellement substitué par hydroxyle ou $C_{1-2}$ alcoxy; $C_{1-2}$alcoyle ou $C_{1-2}$alcoxy halogéno-substitué; $S(O)_nR^2$ où n représente un entier 0, 1 ou 2 et $R^2$ représente $C_{1-2}$alcoyle éventuellement substitué par hydroxyle ou $C_{1-2}$alcoxy; ou bien Ar est éventuellement substitué par un radical $NR^3R^4$ ne comptant pas plus de 5 atomes de carbone où $R^3$ et $R^4$ sont identiques ou différents et représentent chacun un radical $C_{1-3}$alcoyle ou bien $NR^3R^4$ forme un cycle hétérocyclique de cinq ou six chaînons contenant éventuellement un ou deux hétéroatomes supplémentaires;

$R^1$ ne compte pas plus de huit atomes de carbone et représente un radical

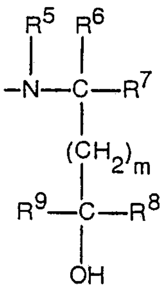

ou

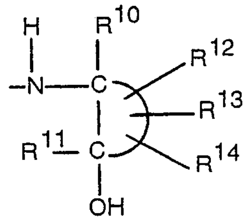

où m représente 0 ou 1;

$R^5$ représente hydrogène ou méthyle;

$R^6$ et $R^7$ sont identiques ou différents et représentent chacun hydrogène ou $C_{1-3}$alcoyle éventuellement substitué par hydroxyle;

$R^8$ et $R^9$ sont identiques ou différents et representent chacun hydrogène ou $C_{1-3}$alcoyle;

$-\overset{\frown}{C-C}-$ représente un cycle carbocyclique saturé de cinq ou six chaînons;

$R^{10}$ représente hydrogène, méthyle ou hydroxyméthyle;

$R^{11}$, $R^{12}$ et $R^{13}$ sont identiques ou différents et représentent chacun hydrogène ou méthyle;

$R^{14}$ représente hydrogène, méthyle, hydroxyle ou hydroxyméthyle;

et avec la restriction que lorsque Ar représente 6-chrysényle, 3- ou 7-fluoranthényle, 2-triphénylényle, ou 1- ou 9-anthracényle éventuellement substitué par un ou deux substituants tels que définis ci-dessus autres que butyle ou butoxy éventuellement substitués; $R^6$ représente $C_{1-3}$alcoyle éventuellement substitué par hydroxyle; $R^7$ représente hydrogène, $C_{1-3}$alcoyle ou hydroxyméthyle; $R^8$ à $R^{12}$ et $R^{14}$ sont tels que définis ci-dessus; $R^{13}$ représente hydrogène et m représente 0, alors $R^5$ représente méthyle, et que le N-(2-hydroxyéthyl)-N-méthyl-9-aminoéthylanthracène est exclu.

2. Composé suivant la revendicaation 1, dans lequel Ar représente 6-chrysényle, 3- ou 7-fluoranthényle, 2-triphénylényle, 1- ou 9-anthracényle, 3-pérylényle, 9-phénanthrényle ou 1-pyrényle.

3. Composé suivant la revendication 1 ou 2, dans lequel $R^1$ représente

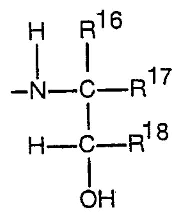

ou

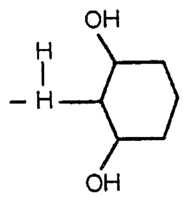

où $R^{13}$ représente $CH_2OH$, $CH(CH_3)OH$ ou $CH_2CH_2OH$,

$R^{17}$ représente hydrogène, $C_{1-3}$alcoyle ou $CH_2OH$,

$R^{18}$ représente hydrogène ou méthyle.

4. Composé suivant l'une quelconque des revendications 1 à 3, dans lequel $R^1$ représente

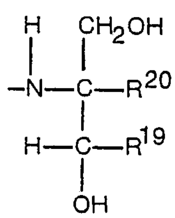

où $R^{19}$ représente hydrogène ou méthyle et

$R^{20}$ représente hydrogène, méthyle ou éthyle.

5. Composé suivant l'une quelconque des revendications 1 à 4, choisi parmi

le 2-méthyl-2-((1-pyrénylméthyl)amino)propanol,
le 3-((1-pyrénylméthyl)amino)propanol,
le 3-méthyl-2-((1-pyrénylméthyl)amino)butanol,
le 2-éthyl-2-((1-pyrénylmethyl)amino)-1,3-propanediol,
le 2-((1-pyrénylméthyl)amino)butanol,
le 2-((1-pyrénylméthyl)amino)propanol,
le 1-((1-pyrénylméthyl)amino)-2-propanol,
le 2-méthyl-2-((1-pyrénylméthyl)amino)-1,3-propanediol,
le 1-((1-pyrénylméthyl)amino)-2-butanol,
le 2-(*N*-(1-pyrénylmethyl)méthylamino)éthanol,
le 2-hydroxyméthyl-2-((1-pyrénylméthyl)amino-1,3-propanediol,
le 2-((1-pyrénylméthyl)amino)-1,3-propanediol,
le 2-(((1-pyrénylméthyl)amino)méthyl)-2-propanol,
le 2-((1-pyrénylméthyl)amino)éthanol,
le 4-((1-pyrénylméthyl)amino)-2-butanol,
le 2-méthyl-2-((9-phénanthrénylméthyl)amino)-1,3-propanediol,
le 2-méthy-2-((3-pérylénylméthyl)amino)-1,3-propanediol,
le 2-méthyl-2-((4-phénanthrénylméthyl)amino)-1,3-propanediol,
le 2-méthyl-2-(((4,5,10-trichloro-9-anthracényl)méthyl)amino)-1,3-propanediol,
le 2-(((10-chloro-2,3-diméthyl-9-anthracényl)méthyl)amino)-2-méthyl-1,3-propanediol,
le 2-(((2-*t*-butyl-10-chloro-9-anthracényl)méthyl)amino)-2-méthyl-1,3-propanediol,
le 2-(((3-*t*-butyl-10-chloro-9-anthracényl)méthyl)amino)-2-méthyl-1,3-propanediol,

le 2-méthyl-2-((2-phénanthrénylméthyl)amino)-1,3-propanediol,
le 2-méthyl-2-((3-phénanthrénylméthyl)amino)-1,3-propanediol,
le 2-((2-anthracénylméthyl)amino)-2-méthyl-1,3-propanediol,
le 2-méthyl-2-(((2,6,10-trichloro-9-anthracényl)méthyl)amino)-1,3-propanediol,
le 2-(((6-bromo-1-pyrényl)méthyl)amino)-2-méthyl-1,3-propanediol,
le 2-(((8-bromo-1-pyrényl)méthyl)amino)-2-méthyl-1,3-propanediol,
le 2-méthyl-2-((4-pyrénylméthyl)amino)-1,3-propanediol,
le 2-((6-chrysénylméthyl)amino)-2-méthyl-1-propanol,
le 2-(((10-butoxy-9-anthracényl)méthyl)amino)-2-méthyl-1,3-propanediol,
le 2-(((10-butyl-9-anthracényl)méthyl)amino)-2-méthyl-1,3-propanediol,
le 2-((3-fluoranthénylméthyl)amino)-2-méthyl-1-propanol,
le 2-((8-fluoranthénylméthyl)amino)-2-méthyl-1,3-propanediol,
le (+−)(2*R**,3*S**)-2-((1-pyrénylméthyl)amino)2-méthyl-1,3-butanediol,
le (+−)(2*R**,3*S**)-2-((9-phénanthrénylméthyl)amino)-2-méthyl-1,3-butanediol,
le 2-(((3-chloro-8-fluoranthényl)méthyl)amino)-2-méthyl-1,3-propanediol,
le 2-(((4-chloro-8-fluoranthényl)méthyl)amino)-2-méthyl-1,3-propanediol,
le 2-éthoxyméthyl-2-((1-pyrénylméthyl)amino)-1,3-propanediol,
le 2-éthoxymethyl-2-((9-phénanthrénylméthyl)amino)-1,3-propanediol,
le 2-(((10-éthoxy-9-phénanthrényl)méthyl)amino)-2-méthyl-1,3-propanediol,
le 2-(*N*-((6-chrysénylméthyl)-*N*-méthylamino)-2-méthyl-1,3-propanediol,
le 2-((3-chrysénylméthyl)amino)-2-méthyl-1,3-propanediol,
le 2-((2-chrysénylméthyl)amino)-2-méthyl-1,3-propanediol,
le 2-(*N*-(3-fluoranthénylméthyl)-*N*-méthylamino)-2-méthyl-1,3-propanediol,
le 3-méthoxy-2-méthyl-2-((1-pyrénylméthyl)amino)-1-propanol,
le 3-méthoxy-2-méthyl-2-((9-phénanthrénylméthyl)amino)-1-propanol,
le 2-(((3-éthyl-8-fluoranthényl)méthyl)amino)-2-méthyl-1,3-propanediol,
le 1-(((4-éthyl-8-fluoranthényl)méthyl)amino)-2-méthyl-1,3-propanediol,
le 2-(((9-éthoxy-1-phénanthrényl)méthyl)amino)-2-méthyl-1,3-propanediol,
le 2-((3-pérylénylméthyl)amino)-3-méthoxy-2-méthyl-1-propanol,
le (+−)(2*R**3*S**)-2-((3-pérylénylméthyl)amino)-2-méthyl-1,3-butanediol,
le 2-((3-pérylénylméthyl)amino)-2-éthoxyméthyl-1,3-propanediol,
le 2-méthyl-2-((9-phénanthrénylméthyl)amino)-1,4-butanediol,
le (1α,2β,3α)-2-((1-pyrénylméthyl)amino)-1,3-cyclohexanediol,
le 2-isopropyl-2-((3-pérylénylméthyl)amino)-1,3-propanediol,
le 2-méthyl-2-((3-pérylénylméthyl)amino)-1,4-butanediol,
le (1α,2β,3α)-2-((3-pérylénylméthyl)amino)-1,3-cyclohexanediol,
le 2-(((3-éthyl-1-pyrényl)méthyl)amino)-2-méthyl-1,3-propanediol,
le 2-isopropyl-2-((9-phénanthrénylméthyl)amino)-1,3-propanediol,
le (1α,2β,3α)-2-((9-phénanthrénylméthyl)amino)-1,3-cyclohexanediol,
le 2-isopropyl-2-((1-pyrénylméthyl)amino)-1,3-propanediol,
le 2-(((8-éthoxy-1-pyrényl)méthyl)amino)-2-méthyl-1,3-propanediol,
et leurs éthers, esters et sels.

6. Chlorhydrate, méthanesulfonate, éthanesulfonate, lactate, citrate ou iséthionate salin d'un composé suivant l'une quelconque des revendications 1 à 5.

7. Composition pharmaceutique comprenant un composé suivant l'une quelconque des revendications 1 à 6 conjointement avec un excipient pharmaceutiquement acceptable.

8. Composition pharmaceutique suivant la revendication 7 se prêtant à l'administration par voie parentérale, qui comprend une composition aqueuse stérile d'un composé suivant l'une quelconque des revendications 1 à 6 qui est isotonique avec le sang du receveur.

9. Procédé pour préparer une composition pharmaceutique suivant la revendication 7 ou 8, qui comprend la mise en association d'un composé de formule (I) ou d'un éther, ester ou sel pharmaceutiquement acceptable de celui-ci avec un excipient pharmaceutiquement acceptable.

10. Composé suivant l'une quelconque des revendications 1 à 6 à utiliser en médicine.

11. Procédé de préparation d'un composé suivant l'une quelconque des revendications 1 à 6, qui comprend

1) la réduction d'un composé de formule (II)

($R^5$ = H dans $R^1$)

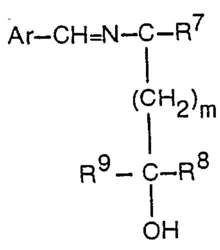

(II)

où $R^1$—$R^4$ sont tels que définis ci-dessus, ou d'un dérivé convenablement protégé de celui-ci, suivie de la déprotection lorsqu'il y a lieu;

2) la réduction d'un composé de formule (V)

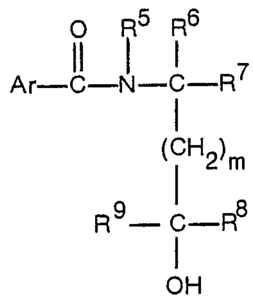

(V)

où Ar et $R^1$—$R^4$ sont tels que définis ci-dessus, et les radicaux hydroxyle sont éventuellement protégés, suivie de la déprotection des radicaux hydroxyle lorsqu'il y a lieu;

3) la réaction d'un composé

$$ArCH_2L$$

(où Ar est tel que défini ci-dessus et L représente un radical partant)

avec un composé de formule (IV)

($R^5$ = H dans $R^1$)

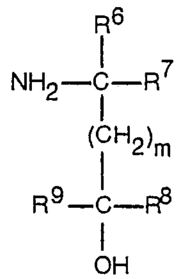

(IV)

où Ar et $R^1$—$R^4$ sont tels que définis en (I), sauf que $R^5$ = H.

12. Intermédiaires chimiques qui sont des 9-anthracénylcarbaldéhydes substitués par un, deux ou trois substituants ne comptant pas plus de 4 atomes de carbone au total lorsqu'ils sont pris ensemble, qui sont identiques ou différents et sont choisis parmi les radicaux $C_{1-4}$-alcoyle et $C_{1-4}$alcoxy, chacun éventuellement substitués par hydroxyle ou $C_{1-2}$alcoxy.